Europäisches Patentamt

European Patent Office

Office européen des brevets

(11) Numéro de publication: **0 258 118 B1**

# (12) FASCICULE DE BREVET EUROPEEN

(45) Date de publication du fascicule du brevet: **18.10.95**

(51) Int. Cl.⁶: **C12N 15/00**, C12N 1/20, C12P 21/02, C12N 9/02, //(C12N1/20,C12R1:19), (C12N9/02,C12R1:19), (C12P21/02,C12R1:19)

(21) Numéro de dépôt: **87401815.3**

(22) Date de dépôt: **05.08.87**

(54) **Procédé de stabilisation d'un plasmide contenu dans une souche bactérienne et souche obtenue.**

(30) Priorité: **05.08.86 FR 8611311**
**15.07.87 FR 8709935**

(43) Date de publication de la demande:
**02.03.88 Bulletin 88/09**

(45) Mention de la délivrance du brevet:
**18.10.95 Bulletin 95/42**

(84) Etats contractants désignés:
**AT BE CH DE ES FR GB GR IT LI LU NL SE**

(56) Documents cités:
EP-A- 0 086 139    EP-A- 0 106 542
EP-A- 0 146 462    EP-A- 0 158 564
EP-A- 0 169 114    EP-A- 0 185 512
FR-A- 2 511 032    GB-A- 2 177 097

CHEMICAL ABSTRACTS, vol. 104, no. 9, 3 mars 1986, page 165, résumé no. 63013t,Columbus, Ohio, US; D.W. LEUNG et al.: "Nucleotide sequence of the partition-function of Escherichia coli plasmid ColE1", & DNA 1985, 4(5), 351-5

(73) Titulaire: **TRANSGENE S.A.**
**11, rue de Molsheim**
**F-67000 Strasbourg (FR)**

(72) Inventeur: **Degryse, Eric**
**6, rue d'Oslo**
**F-67000 Strasbourg (FR)**

(74) Mandataire: **Schrimpf, Robert et al**
**Cabinet Regimbeau**
**26, Avenue Kléber**
**F-75116 Paris (FR)**

## Description

L'application pratique de la technologie de l'ADN recombinant à la production de molécules intéressantes par des microorganismes se heurte généralement au problème de l'instabilité des plasmides recombinants.

Les vecteurs de clonage et d'expression couramment utilisés en laboratoire sont généralement des plasmides multicopies et leur transmission stable à la descendance est assurée par le grand nombre de plasmides par génome cellulaire (Jones et al. 1980). Toutefois, l'introduction de gènes étrangers dans ces plasmides entraîne des degrés divers d'instabilité au cours des cycles de multiplication des bactéries. Or, des productions industrielles peuvent nécessiter des cultures de 1 000 litres, aboutissant à plus de $10^{16}$ cellules, après plus de 50 générations. Il est donc essentiel de stabiliser les plasmides dans les bactéries pour assurer leur présence et donc l'expression de la molécule étrangère, jusqu'à la fin de la culture en fermenteur.

La stabilisation des plasmides par intégration d'un gène codant pour la résistance à un antibiotique est une pratique classique de laboratoire mais qui ne peut être appliquée à l'usage industriel pour diverses raisons :
- l'utilisation d'une souche de bactérie résistante à un antibiotique peut représenter un risque pour l'environnement ;
- la quantité d'antibiotique nécessaire pendant la culture augmente considérablement le coût de la production ;
- l'utilisation d'antibiotique ne peut être envisagée pour la production de substances à usage thérapeutique humain ou vétérinaire.

Il est donc essentiel de mettre au point d'autres méthodes de sélection des bactéries portant un plasmide recombinant. Les quelques modèles qui ont déjà été appliqués reposent sur le même principe : la cellule hôte est mutée (mutation auxotrophe ou introduction d'un gène létal pour la bactérie) de manière à empêcher sa multiplication en l'absence d'un plasmide codant pour un caractère qui complémente la déficience de l'hôte.

Par exemple, Skogman et Nilson (1984 et 1985) ont utilisé la complémentation d'une mutation thermosensible val S par le gène val S porté par un plasmide ; dans ce modèle, la stabilité du plasmide, qui porte entre l'opéron tryptophane, est totale après 200 générations à température non permissive, alors qu'on observe 1,2 % de perte du plasmide à chaque génération en conditions non sélectives (30 °C).

Miwa et al. (1984) ont utilisé comme souche hôte un mutant de E. coli streptomycine-dépendant ($Sm^d$) et un plasmide portant un gène (rpsl d'une souche $Sm^R$) qui masque le phénotype $Sm^d$ et rend la souche indépendante de la streptomycine en assurant une stabilité du plasmide supérieure à 99 %.

Herthberger et Rosteck (1984) ont rendu une bactérie lysogène pour un prophage lambda délété de son répresseur ; la cellule ne peut échapper à la lyse qu'en présence d'un plasmide portant le répresseur lambda cl.

On a développé un nouveau modèle de sélection basé sur la complémentation d'une mutation chromosomique $dap^-$ par un gène plasmidique $dap^+$, ce qui assure la survie des seules cellules qui portent le plasmide.

L'acide diaminopimélique (DAP) est un composant de la paroi des bactéries ; il constitue aussi un intermédiaire de la biosynthèse de la lysine à partir d'aspartate. Une souche déficiente en une enzyme de la biosynthèse du DAP ne sera pas capable de se multiplier en milieu minimum ; l'addition de lysine dans le milieu permettra la croissance mais entraînera rapidement la lyse de la cellule qui n'incorpore pas de DAP dans sa membrane (Work 1950 - Davis et al., 1973).

La déficience en une enzyme de la biosynthèse du DAP peut être complémentée par l'introduction du gène correspondant sur un plasmide et, en particulier, sur le vecteur d'expression de la protéine étrangère dont on veut assurer la production. Si la bactérie perd le plasmide, elle redevient $dap^-$ et ne peut plus se multiplier. Ce modèle présente l'avantage de pouvoir s'appliquer à tout milieu de culture dépourvu de DAP, c'est-à-dire tout milieu industriel ce production et tout milieu riche ou minimum additionné de lysine.

Le système présente aussi l'avantage de ne pas perturber la synthèse de l'ADN, de l'ARN ou des protéines.

On a construit une souche $dapD^-$ (délétée d'un des 9 gènes de la voie biosynthétique de la lysine, le gène D, correspondant à la tétrahydropicolinate-N-succinyl transférase) et introduit le gène dapD sur un plasmide d'expression couramment utilisé (portant le gène à exprimer sous le contrôle du promoteur $P_L$). En condition de sélection, on peut montrer que le plasmide est stable pendant au moins 150 générations.

Le clonage d'un autre gène (dapA) codant pour une enzyme de la biosynthèse de la lysine sur un plasmide a déjà été réalisé, dans le but d'augmenter la production de lysine (Dauce-Le Reverend et al.

2

1982) mais ces auteurs ne maîtrisent pas la stabilité de leur plasmide.

Le gène dapD de E. coli a déjà été cloné dans le plasmide pBR322 et sa séquence nucléotidique a été publiée par Richaud et al. (1984) mais le but de ce travail est seulement l'étude de la régulation de ce gène.

la présente invention concerne un procédé de stabilisation d'un vecteur plasmidique contenu dans une bactérie, caractérisé en ce que la bactérie comporte une mutation chromosomique dap⁻ et en ce que le vecteur plasmidique porte un gène dap⁺.

Parmi les mutations chromosomiques dap⁻, on utilisera, de préférence dapD⁻ mais d'autres gènes codant pour une enzyme de la biosynthèse du DAP pourraîent être utilisés à condition de prévoir un plasmide vecteur portant le gène correspondant ; dans le cas d'une souche dapD⁻, le gène à insérer dans le plasmide sera dapD.

C'est pourquoi la présente invention a plus precisément pour objet une souche bactérienne comportant une mutation du gène dapD et transformée par un vecteur plasmidique portant un gène dapD intact, ainsi qu'un gène codant pour une proteine d'intéret, et les éléments assurant son expression dans la bactérie hôte.

Le système de stabilisation dap n'interfère pas avec l'expression de la protéine, il peut donc être utilisé avec un vecteur d'expression quelconque,

Afin d'éviter les recombinaisons homologues possibles entré le plasmide dapD⁺ et le gène dapD muté du chromosome, il est préférable de prévoir une délétion importante du gène dapD du chromosome.

la présente invention concerne plus particulièrement une souché telle que décrite précédemment, caractérisée en ce que la mutation chromosomique dap est une délétion d'une partie au moins du gène dapD et en ce que le vecteur plasmidique porte un gène dapD intact.

Il est clair qu'afin d'éviter toute recombinaison, une délétion totale du gène dapD sera la solution la plus satisfaisante.

Toutefois, un système de sélection si puissant soit-il ne peut remédier à l'instabilité propre d'un plasmide. C'est pourquoi, afin d'augmenter la stabilité des vecteurs d'expression en cause par des moyens génétiques, il est possible d'introduire dans lesdits vecteurs une séquence qui les maintient à l'état de monomères, en particulier la séquence "cer".

Le "cer" (Summers et Sherratt, 1984) est un élément qui ne code pour aucune protéine et dont la présence favorise le maintien monomérique d'un plasmide. Si le plasmide se multimérise, le nombre d'unités distribuables aux cellules filles chute et on obtient plus facilement des cellules dépourvues de plasmide. Indirectement donc, le cer stabilise le plasmide en le maintenant à l'état de monomère.

C'est pourquoi la présente invention concerne également des vecteurs plasmidiques qui comportent en outre le gène codant pour une protéine d'intérêt industriel et les éléments assurant son expression dans la bactérie hôte, et, notamment, le gène codant pour une hirudine ou l'un de ses variants naturels ou synthétiques, le $C_{2,3}O$, l'interféron gamma ou l'alpha antitrypsine par exemple, ainsi que les souches transformées par ces différents vecteurs et les procédés de préparation des protéines industrielles par culture desdites souches transformées sur milieu complet et récupération de la protéine après culture.

Dans ce qui suit, le système d'expression comprend le promoteur de gauche du phage lambda$P_L$, mais il pourraît s'agir d'un autre promoteur actif dans la bactérie en cause. Ceci explique pourquoi il ne sera pas procédé à une description complète des éléments d'expression de la protéine hétérologue, ce type de plasmide étant maintenant largement connu. Il conviendra simplement d'insérer le gène dapD ou autre dans un site non essentiel du vecteur plasmidique.

Les plasmides en cause peuvent être utilisés pour transformer n'importe quelle souche E. coli rendue dapD⁻.

Grâce au procédé selon l'invention, on obtient des souches bactériennes dans lesquelles les plasmides d'expression sont stables en milieu complet sans besoin d'une pression sélective par un antibiotique ou d'un milieu particulier dépourvu d'un acide aminé par exemple. De plus, le procédé selon l'invention exerce une contre-sélection contre les bactéries qui ont perdu le plasmide.

L'invention concerne également un procédé pour la préparation d'une protéine d'intérêt industriel à partir d' une bactérie, dans lequel on cultive une bactérie notamment selon l'invention, et on récupère ladite proteine.

Les exemples ci-après ont pour but de démontrer la stabilité de plasmides portant le gène dapD dans des bactéries dap⁻ en comparaison avec des plasmides portant le gène Amp^r.

Ces plasmides portant le gène dapD ont en outre été utilisés pour exprimer les gènes codant pour l'hirudine et l'interféron gamma.

Ces deux gènes sont placés sous le contrôle du promoteur $P_L$ du phage lambda et les souches hôtes de E. coli contiennent le répresseur thermosensible CI857 ; ce système permet d'induire l'expression du gène contrôlé par $P_L$ par élévation de la température.

La souche de E. coli TGE 900 qui comporte le répresseur CI857 a été modifiée pour devenir dapD⁻ en donnant la souche TGE 7615 ou TGE7214 et la souche N 5969 a été modifiée pour devenir TGE7303.

Les plasmides suivants ont ensuite été préparés selon les schémas ci-annexés :

| | Amp^r | dapD | Protéine étrangère |
|---|---|---|---|
| Figure 1 - pTG764 | + | + | 0 |
| Figure 2 - pTG720 | + | 0 | Hirudine |
| Figure 2 - pTG771 | + | + | Hirudine |
| Figure 3 - pTG775 | 0 | + | Hirudine |
| Figure 3 - pTG776 | 0 | + | Hirudine |
| Figure 4 - pTG766 | 0 | + | 0 |
| Figure 4 - pTG767 | 0 | + | 0 |
| Figure 5 - pTG7671 | 0 | + | IFN gamma |

Figure 6 : Carte de restriction du fragment HindIII-BamHI du pDB6.

Figure 7 : Schéma des inserts PstI du pDB6 dans M13mp8 et position des sites EcoRI qui y sont introduits.

Figure 8 : Carte de restriction du fragment KpnI-BglII du pTG47.

Figure 9 : Démonstration de la délétion du gène dap dans les ADNs chromosomiques de différentes souches, par autoradiographie de "Southern blots".

Figure 9A :

. sonde = gène kan^R porté par le fragment EcoRI du pTG47
. bandes
6 à 9 ADN coupé par PstI
10 à 14 ADN coupé par BamHI et HindIII
. bandes
6 et 10 = souches GC4540
7 et 11 TGE7213
8 et 12 TGE7214
9 et 13 TGE901
14 pDB6
15 marqueurs de poids moléculaires

Figure 9B :

. sonde = côté 5' du gène dapD porté par le fragment EcoRI du M13TG620
. bandes
4 à 7 ADN coupé par PstI
8 à 11 ADN coupé par BamHI et HindIII
. bandes
4 et 8 = souches GC4540
5 et 9 TGE7213
6 et 10 TGE7214
7 et 11 TGE901
. bandes
13 marqueurs de poids moléculaires
1 et 12 pDB6 coupé par PstI ou BamHI et HindIII
2 M13TG620 coupé par PstI
3 M13TG597 coupé par PstI

Figure 9C :

. sonde = régions flanquantes du gène dapD portées par le fragment KpnI-BglII du pTG47. ADNs chromosomiques coupés par PstI.
bandes
4 = souches GC4540
5 TGE7213
6 TGE7214
7 TGE901

. bandes

8 = marqueurs de poids moléculaires

1 fragment BamHI-HindIII de pDB6 coupé par PstI

2 M13TG620 coupé par PstI

3 M13TG597 coupé par PstI

Figure 9D :

. sonde = régions flanquantes du gène dapD portées par le fragment KpnI-BglIII du pTG47.

. ADNs chromosomiques coupés par PstI

. bandes

4 = souches RH5345

5 RL58

6 TGE7615

1, 2, 3 (comme en 9C )

7 = marqueurs de poids moléculaire

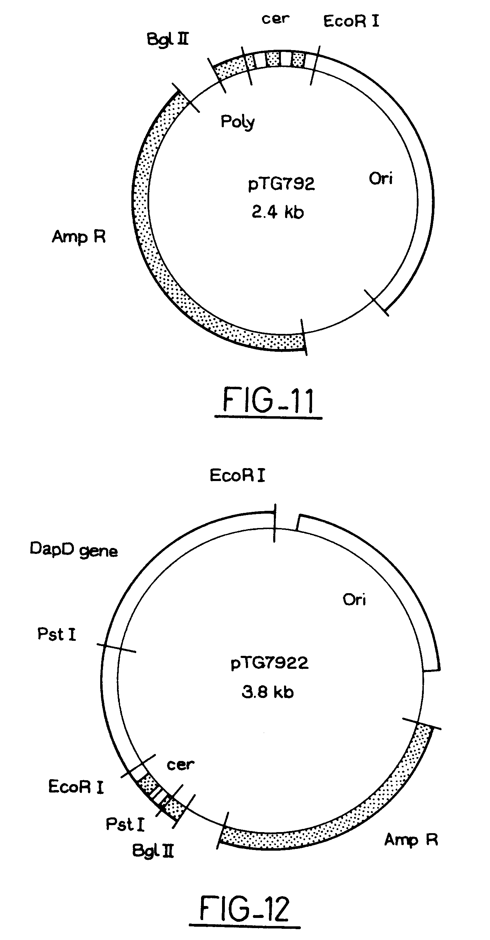

Figure 10 :       Schéma et carte de restriction du pTG790.

Figure 11 :       Schéma et carte de restriction du pTG792.

Figure 12 :       Schéma et carte de restriction du pTG7922.

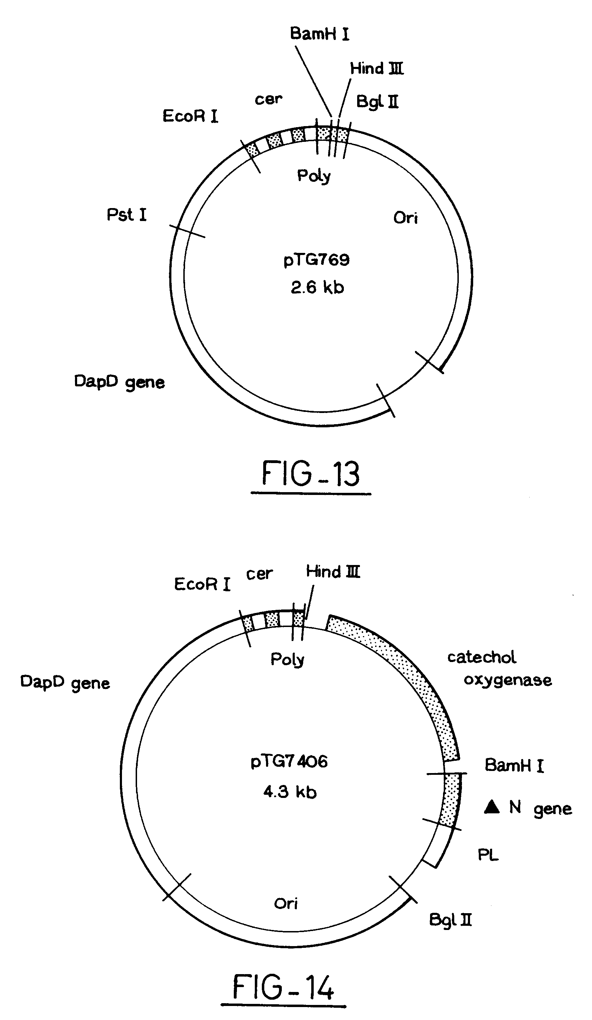

Figure 13 :       Schéma et carte de restriction du pTGT69.

Figure 14 :       Schéma et carte de restriction du pTG7406.

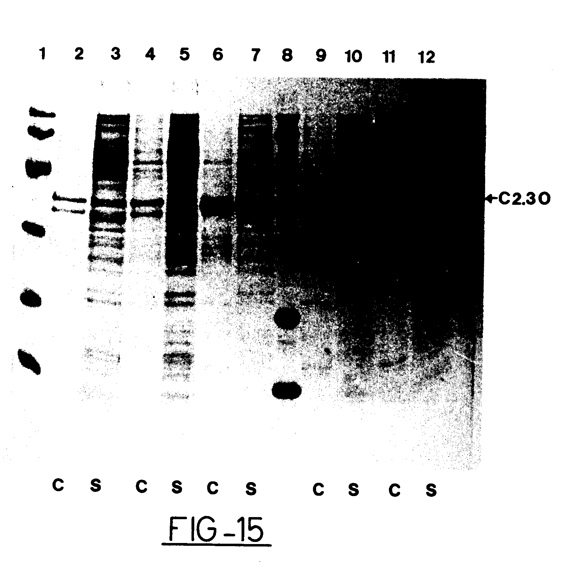

Figure 15 :       Electrophorèse sur gel d'acrylamyde-SDS, révélé au bleu de Coomassie, des protéines synthétisées par TGE7213/pTG7407.

. bandes 1 et 8 = marqueurs de poids moléculaires

. bandes 2 à 5 = culture à 30°C, 4 heures (2 et 3) ou 7 heures (4 et 5)

. bandes 6 à 12 : culture induite à 42°C, 4 heures (6, 11 et 12) ou 7 heures (7, 9 et 10)

C = fraction insoluble ; 20 $\mu$l

S = fraction soluble ; 40 $\mu$l

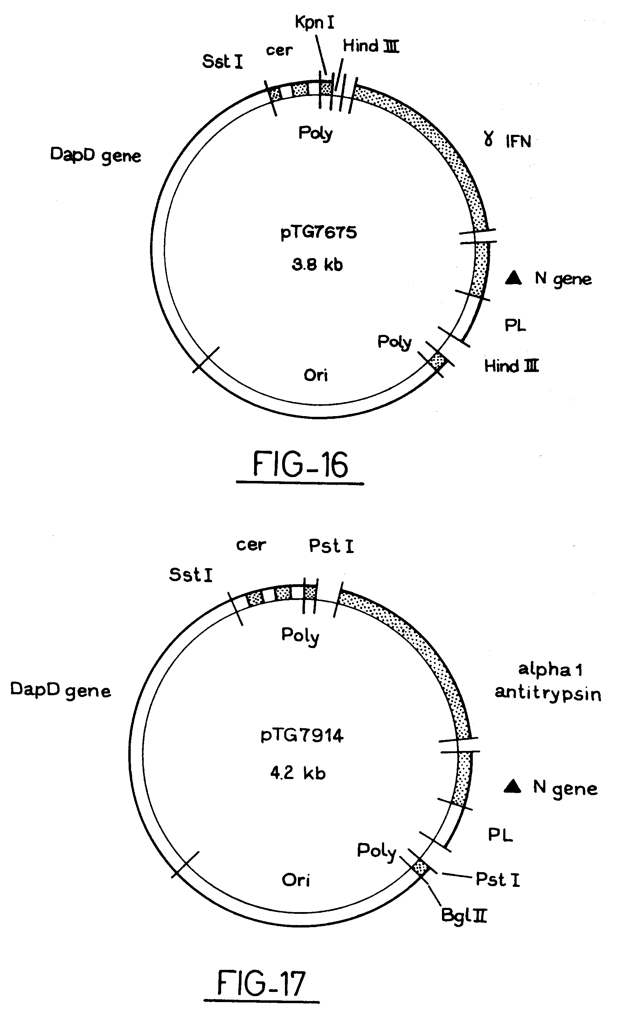

Figure 16 :       Schéma et carte de restriction du pTG7675.

Figure 17 :       Schéma et carte de restriction du pTG7914.

## METHODES

Les différentes enzymes sont, sauf indication contraire, utilisées selon les techniques connues.

## Transformation

Les cellules compétentes ont été préparées selon la méthode de Hanahan (1983). Leur compétence s'élève à $10^4$ à $10^5$ transformants/$\mu$g ADN pour la souche RL58 (Bollen et al. 1979) et $10^5$ à $10^6$ transformants/$\mu$g ADN pour les souches TGE7615, TGE7214 ou TGE 7303.

De manière générale, on ajoute à O,2 ml d'un stock de cellules compétentes 1 à 10 $\mu$l d'une solution ayant la dilution appropriée, d'une préparation d'ADN contenant le plasmide que l'on veut introduire dans la souche. On laisse réagir les cellules avec l'ADN pendant 15 minutes à 0°C puis on les incube à 37°C pendant 90 secondes. On les remet à 0°C pendant 5 minutes puis on y ajoute 0,8 ml de milieu LB. Pour la souche RL58 ou pour la souche TGE 7615, ce milieu doit contenir du DAP à raison de 8 gamma /ml. Ceci est nécessaire à la croissance des cellules dapD⁻, même celles qui contiennent le plasmide portant le gène dapD. On incube les cellules de manière générale à 30°C avec une forte agitation (toutefois, on peut les mettre à 37°C si le plasmide ne contient pas le promoteur $P_L$). Le temps d'incubation est de 6O minutes, mais il peut être prolongé à 90 minutes pour une croissance à 30°C pour la souche TGE 7615.

Ensuite, des volumes fixes (0,1 ml) sont étalés sur du milieu LB solide. Pour les plasmides contenant le gène de résistance à l'ampicilline, on sélectionne les clones par addition de 100 gamma/ml d'ampicilline ; les clones contenant le gène dapD sont sélectionnés, à partir d'une culture qui est dapD⁻ dans son ensemble, sur le milieu LB sans autres additions. Les boîtes sont incubées pendant 24 heures à 30°C. Les colonies sont ensuite repiquées au cure-dents dans 3 ml de milieu LB et après une nuit de croissance à 30°C le plasmide est isolé et sa structure confirmée sur gel d'agarose, après digestion par les enzymes de restriction appropriées.

Les plasmides contenant le gène dapD peuvent être transformés dans n'importe quel E. coli qui porte la mutation dapD⁻ (par exemple RL58, TGE7615 , TGE7214 ou TGE7303) ; si, en outre, ils contiennent le promoteur $P_L$ du bactériophage lambda, ils doivent nécessairement être transformés dans des souches de E. coli dapD⁻ qui expriment le répresseur C1857 du bactériophage lambda, soit sur un plasmide, soit dans un chromosome, ou le Cl et une mutation Rec (par exemple RecA441).

EXEMPLE 1

Introduction d'un gène muté dapD⁻ dans la souche E. coli TGE900

La souche hôte E. coli TGE900 a été rendue dap⁻ par une conjugaison avec un mutant dapD⁻ connu, RL58.

La souche TGE900 est un dérivé de la souche N4830 décrite par Gottesman et al. (1980) dont les caractéristiques sont : su⁻ F⁻ his ilv bio (λcl857 Δ Bam ΔHI) Sm'. Elle est couramment utilisée comme hôte pour les vecteurs d'expression dans lesquels le gène étranger est placé sous le contrôle du promoteur $P_L$ de lambda parce qu'elle permet d'induire l'expression à volonté par une augmentation de la température (supérieure à 37°C) ce qui inactive le répresseur thermosensible (lambda cl857) porté par la bactérie.

La souche dap⁻ RL58 (met B dapD₂ met D279 Hfr P₄ X) a été décrite par Bollen et al. (1979).

A partir de cette souche, on a sélectionné un mutant spontané résistant au triméthoprim (après étalement de la souche sur une boîte contenant 2 gamma/ml de triméthoprim et confirmation du caractère résistant d'une colonie choisie en milieu contenant 4 gamma/ml de triméthoprim). En effet, le gène codant pour cette résistance est suffisamment proche de la mutation dapD⁻ pour que si la souche réceptrice après conjugaison, devient résistante au triméthoprim elle soit également devenue dapD⁻.

La souche résistante a été appelée TGE755 ; ses caractéristiques utiles sont Hfr dapD⁻ Tmp'.

Après conjugaison des souches TGE900 (F⁻ Sm^res dap⁺) et TGE755, interrompue après 15 minutes, par étalement sur milieu minimum contenant du DAP, de la streptomycine et du triméthoprim, on sélectionne des colonies Sm' Tmp' ; on vérifie ensuite le caractère dap⁻ des souches sélectionnées sur milieu LB et la présence des mutations auxotrophes de la souche parentale (his ilv) sur milieu minimum + DAP

Une souche (his ilv dap⁻) a été retenue : TGE7615.

EXEMPLE 2

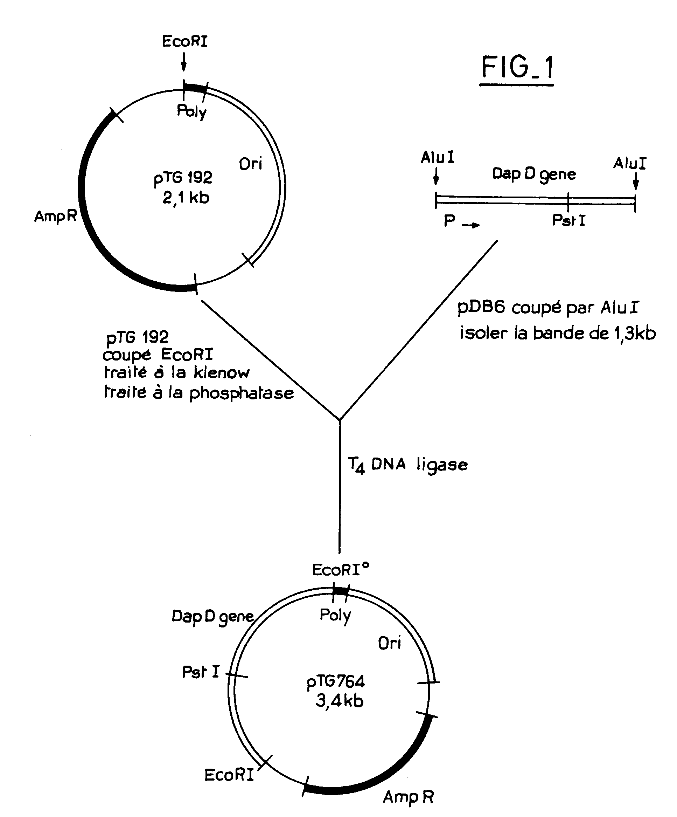

Construction d'un plasmide vecteur portant le gène dapD de E. coli (figure 1)

a) Construction d'un plasmide de clonage à usage multiple comprenant un polylinker à 12 sites de restriction

La construction a été amorcée à partir du plasmide pML₂ (Lusky et Botchan, 1981), dérivé du pBR322 par délétion des nucléotides 1089 à 2491. Ce plasmide a conservé l'origine de réplication de pBR322 et le gène de la beta lactamase (résistance à l'ampicilline).

La séquence de reconnaissance de l'enzyme PstI a été éliminée par insertion d'un fragment AhaIII-AhaIII de pUC8 qui porte une mutation PstI° induite à l'éthane-sulfonate (Vieira et Messing, 1982), entre deux sites AhaIII de pML₂, ce qui délète 19 paires de bases du pML₂. Le plasmide résultant, pTG190, porte la mutation PstI° de pUC8.

Un linker BglII (5'-dCAGATCTG-3' ; Collaborative Research) a été inséré dans le site unique NruI, par la technique de "linker tailing" décrite par Lathe et al., 1984. La construction résultante est le pTG191.

Le pTG191 a été ouvert par EcoRI et BglII (ce qui délète le gène de résistance à la tétracycline) et religué avec le segment EcoRI-BglII du phage M13TG131 (Kieny et al., 1983) qui comprend un polylinker à 12 séquences de reconnaissance d'enzymes de restriction.

Le plasmide résultant est le pTG192.

b) Clonage d'un gène dapD dans le plasmide pTG192

Le gène chromosomique dapD de E. coli a été inséré dans le plasmide pACYC184 pour donner le pDB6 (Bendiak et Friesen, 1981). Ce gène dapD a été récupéré à partir de pDB6 sous forme d'un fragment AluI de 1,3 kb et inséré dans le site EcoRI de pTG192 (les extrémités EcoRI ayant préalablement été réparées par traitement avec le fragment Klenow de l'ADN polymérase I).

Parmi les plasmides résultants, on choisit le pTG764 dans lequel un seul site EcoRI est reconstitué. pTG764 porte donc le gène dapD et le gène de résistance à l'ampicilline du pTG192 (originellement du pUC8).

EXEMPLE 3

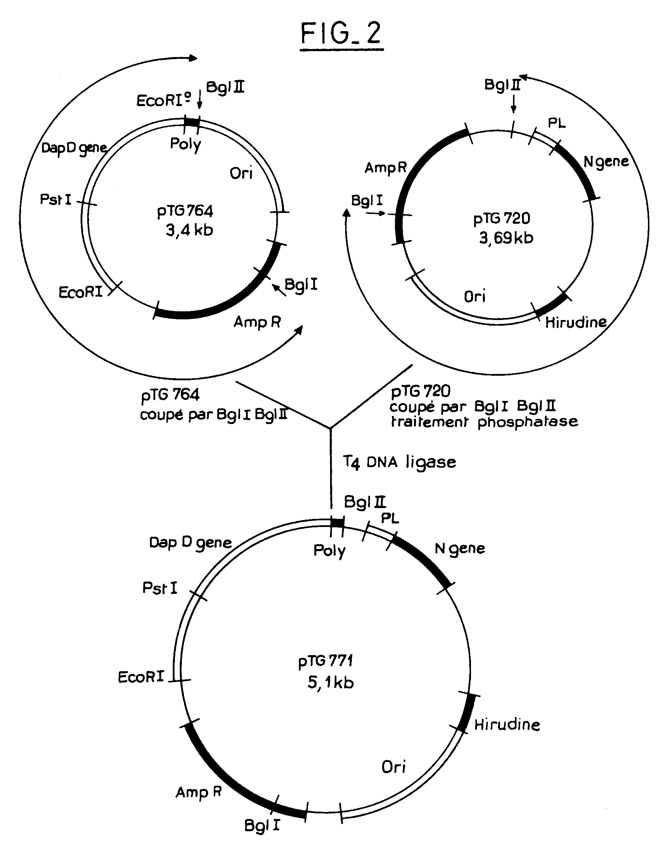

Plasmide d'expression de l'hirudine contenant les gènes dapD et Amp$^r$ (figure 2)

Le plasmide pTG720 décrit dans le brevet français n°84 04755 comporte essentiellement le gène de l'hirudine sous le contrôle du promoteur P$_L$ de lambda.

Le plasmide pTG720 digéré par BglII et BglI donne un fragment de 2,74 kb qui porte le gène de l'hirudine et une partie du gène amp$^r$. Ce fragment, traité à la phosphatase, est religué au fragment BglII-BglI du plasmide pTG764 qui porte le gène dapD et un autre fragment du gène amp$^r$. Le plasmide résultant, pTG771, porte le gène amp$^r$ complet reconstitué, le gène dapD et le gène codant pour l'hirudine.

Les colonies de TGE7615 transformées par ce plasmide peuvent être sélectionnées soit sur milieu LB (sélection pour le caractère dap$^+$) soit sur milieu LB + ampicilline.

Après induction, la souche transformée produit de l'hirudine.

EXEMPLE 4

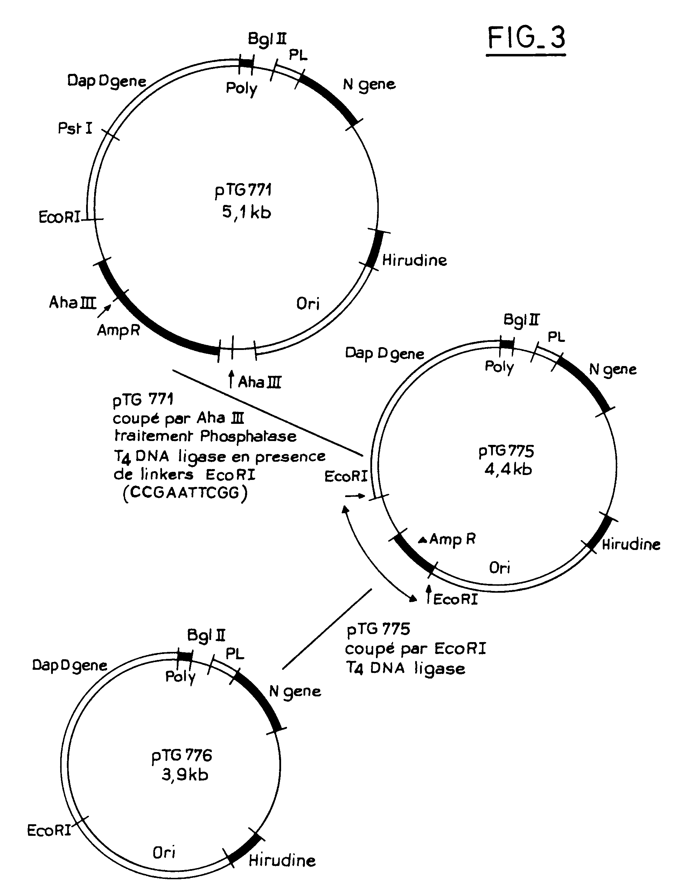

Plasmide d'expression de l'hirudine contenant le gène dapD et ne contenant plus Amp$^r$ (figure 3)

Le plasmide de départ est pTG771.

On effectue une digestion avec AhaIII pour éliminer le petit fragment comportant l'extrémité 3' du gène codant pour Amp$^r$ et on referme le plasmide par une ligation en présence d'un "linker" EcoRI :
CCGAATTCGG

On obtient le plasmide pTG775.

Une digestion EcoRI suivie d'une ligation permet d'éliminer entièrement le gène codant pour Amp$^r$.

On obtient le plasmide pTG776.

Les bactéries TGE7615 transformées par ce plasmide peuvent être sélectionnées en milieu LB, sans addition (sélection pour le caractère dap$^+$).

Après induction, la souche transformée produit de l'hirudine.

EXEMPLE 5

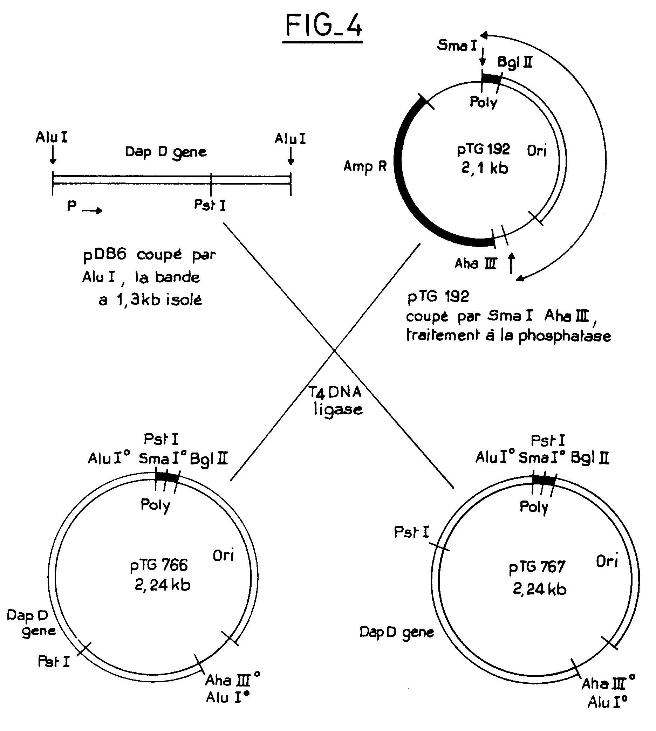

Construction de plasmides portant le gène dapD et ne comportant pas le gène Amp$^r$ (figure 4)

Le plasmide de départ est le plasmide pTG192.

Après digestion par SmaI et AhaIII, puis traitement à la phosphatase, le fragment du vecteur de 0,95 kb est isolé et ligué avec le fragment AluI de 1,3 kb de pDB6 qui porte le gène dapD. Le gène amp$^r$ a donc été complètement délété. On transforme la souche TGE7615 avec ce nouveau plasmide et on sélectionne sur milieu LB pour obtenir des clones qui portent le gène dapD.

On obtient des plasmides qui portent le gène dap dans les deux orientations ; l'orientation de l'insert peut être déterminée par digestion avec PstI. Pour deux candidats choisis :
  . pTG766 libère des fragments de 1,34 kb et 0,9 kb
  . pTG767 libère des fragments de 1,85 kb et 0,4 kb.

EXEMPLE 6

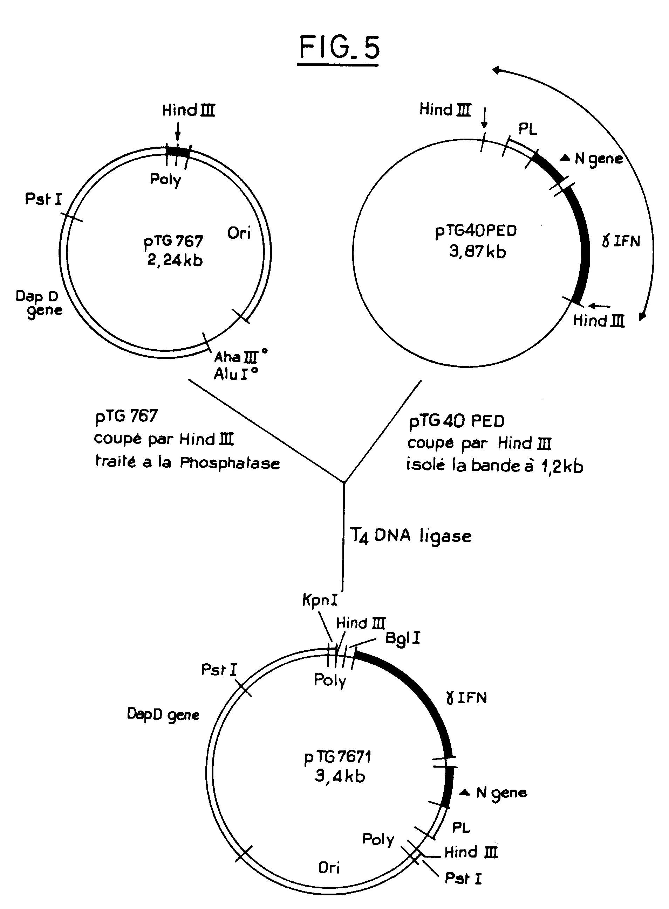

Plasmide d'expression de l'interféron gamma portant le gène dapD (Figure 5)

Le plasmide pTG40 (EP-A-0146462) (PED) porte sur un fragment HindIII de 1,2 kb le gène de l'interféron gamma sous le contrôle du promoteur P$_L$. Après digestion par HindIII, ce fragment est récupéré et inséré dans le site HindIII de pTG767 préalablement traité à la phosphatase. Après ligation, on transforme TGE7615 et on sélectionne pour la présence du gène dapD sur milieu LB. On retient deux plasmides qui portent l'insert dans des orientations opposées :
  . pTGT671 (qui a le P$_L$ proximal de l'origine de réplication et dans la même orientation) et
  . pTG7672.

Les bactéries TGE7615 transformées par ces plasmides peuvent être sélectionnées en milieu LB, sans addition (sélection pour le caractère dap$^+$).

Après induction, la souche transformée produit de l'interféron gamma.

Les exemples suivants sont destinés à mettre en évidence les caractéristiques des souches transformées selon la présente invention.

Tous les plasmides portant le gène dapD se trouvent dans la souche TGE7615, les autres sont dans la souche TGE900.

La stabilité des différents plasmides a été testée sur les milieux appropriés :

- on appellera milieu sélectif le milieu LB pour les plasmides selon l'invention et le milieu LB + ampicilline pour les plasmides pTG720 et pTG40 ;

- on appellera milieu non sélectif le milieu LB + DAP pour les plasmides selon l'invention et le milieu LB pour les autres plasmides.

On effectue une étude de stabilité sur 110 générations, par plusieurs cycles successifs de dilution et multiplication des bactéries. A la fin de cette phase de multiplication, on mesure la stabilité du plasmide de la manière suivante :

- on mesure le nombre de cellules viables dans la culture, par dilution appropriée et comptage des cellules en milieu non sélectif gélosé ; et

- après un temps de croissance, un échantillonnage significatif de ces colonies est repiqué en parallèle sur milieux sélectif et non sélectif gélosés et le pourcentage de colonies ayant perdu le plasmide et donc la capacité de croître sur milieu sélectif est déterminé.

Les résultants obtenus avec les plasmides portant le gène de l'interféron sont présentés dans le tableau 1 :

TABLEAU 1

| Evaluation de la perte du plasmide par les bactéries après 110 générations | | | | |
|---|---|---|---|---|
| Souche | TGE900 | TGE7615 | TGE7615 | TGE7615 |
| Plasmide | pTG40 | pTG766 | pTG767 | pTG7671 |
| Sélection | Amp | dapD | dapD | dapD |
| Milieu LB p-/c/génération * | $2.10^{-3}$ | $< 5.10^{-5}$ | $< 5.10^{-5}$ | $3.10^{-5}$ |
| Milieu LB + DAP | $2.10^{-3}$ | $< 5.10^{-5}$ | $< 5.10^{-5}$ | $3.10^{-5}$ |

* c = nombre de cellules viables p- = cellules ayant perdu le plasmide p-/c/génération = (nombre de p-/nombre de c testées) x nombre de générations

Des essais équivalents réalisés avec les plasmides portant le gène de l'hirudine et avec le vecteur de clonage pTG766, après multiplication des bactéries pendant 170 générations en milieu LB, donnent les résultats suivants :

TABLEAU 2

| Evaluation de la perte du plasmide par les bactéries après 170 générations en milieu LB | | | | |
|---|---|---|---|---|
| Souche | TGE900 | TGE7615 | TGE7615 | TGE7615 |
| Plasmide | pTG720 | pTG771 | pTG776 | pTG766 |
| p + (%) | 96 | 98 | 100 | 100 |
| p-/c/génération | $2.10^{-4}$ | $1,5.10^{-4}$ | $< 1,1.10^{-4}$ | $< 1,1.10^{-4}$ |

Les résultats représentés dans les tableaux 1 et 2 montrent :

1°) que la stabilité des vecteurs dap est améliorée d'un facteur 10 par rapport au plasmide portant le gène amp$^r$ ;

2°) que la différence entre le milieu sélectif et non-sélectif est minime ;

3°) que les vecteurs de clonage pTG766 et pTG767 ont une perte inférieure à $5.10^{-5}$ p-/c/génération ;

4°) que la stabilité du pTG40 est nettement inférieure à celle des vecteurs dap$^+$ mais il convient de préciser que le phénomène se marque surtout après 50 générations ; au début de la culture, la perte n'est que de $2,5.10^{-4}$ p-/c/génération ;

5°) que la perte des plasmides témoins amp$^r$, pTG40 et pTG720, portant respectivement les gènes de l'interféron et de l'hirudine, est du même ordre de grandeur ; cette perte des plasmides au cours du

8

temps est réduite quand ils portent le gène dapD (pTG766, 767, 7671 et 776).

Il faut toutefois remarquer que la stabilité du plasmide portant le gène dapD et le gène de la résistance à l'ampicilline (pTG771) est moins bonne et reste comparable à celle de pTG720. On interprète ce résultat par l'analyse du contenu plasmidique sur gel d'agarose ; celle-ci montre que pTG771 se tétramérise, phénomène qui se répercute sur la partition des plasmides et qui rend leur perte plus élevée (Summers et Sherratt, 1984). Il faut ajouter que sur un nombre réduit de générations (30) ce phénomène de multimérisation ne se produit pas et que sur un total de 70 générations la perte de pTG771 reste inférieure à $2,8.10^{-4}$ p-/c/génération.

Donc les plasmides qui contiennent le gène dapD voient leur stabilité accrue par rapport aux plasmides portant le gène amp$^r$ et leur perte devient minime.

EXEMPLE 7

Stabilité des plasmides à 37 ou 42°C

L'influence de la température sur la stabilité des plasmides ne peut être étudiée, pour ceux qui contiennent un gène codant pour une protéine étrangère placé sous le contrôle du promoteur $P_L$ sans induire l'expression de cette protéine étrangère. Néanmoins, il faut remarquer que dans les conditions où l'on travaille l'induction est suivie par une augmentation de D.O. de 0,3 à 3,6, ce qui correspond à 3,6 générations. Donc, en fait, les bactéries font le plus grand nombre de générations à 30°C, avant l'induction, avec une perte qu'on a déjà déterminée dans l'exemple précédent.

Lorsqu'on induit l'expression d'une protéine étrangère (par exemple l'hirudine pour pTG720 et l'interféron gamma pour pTG40) on constate, après un certain temps (2 à 4 heures), une mortalité de la culture de E. coli à raison de 90 % à 95 %. Il s'agit de cellules contenant le plasmide (puisque la cellule hôte p- est capable de croître à 37 et 42°C). Cette mortalité augmente forcément la proportion des cellules p- par rapport aux cellules en vie d'un facteur de 10 à 20. Lorsque l'ensemble des cellules en vie a fortement diminué et que les cellules p- forment une fraction significative de la population, on mesure la multiplication des cellules p-, ce qui fait diminuer fortement le rapport p+/p- (voir, par exemple, tableau 3, les résultats obtenus après 5 h 30 et 7 heures).

Il est évident que le paramètre p-/cellule/génération a perdu sa signification dans de telles conditions puisque les p+ ne se multiplient plus. On propose le paramètre suivant : p-/ml/unité de densité optique, qui ne mesure que les cellules p- à chaque moment de la culture. En outre, il permet de comparer deux cultures différentes pour leur perte de plasmide. Ce paramètre est calculé selon la formule suivante :

$$p\text{-/ml/DO} = [1-(\%p+/100)] \times c.\ viables/ml/DO.$$

Finalement, si l'on rapporte ce paramètre au nombre total des cellules (qui est de $4.10^8$ dans les conditions présentes) on peut obtenir le pourcentage des cellules (Fp-) dans la culture et estimer le degré de contamination de la culture par des cellules p- à chaque moment. Le Fp-est calculé de la manière suivante :

$$Fp\text{-} = 4.10^8\ c/ml/DO \times 100/(p\text{-/ml/DO}).$$

Fp- est un paramètre objectif qui permet de décider si une culture destinée à la production d'une molécule est suffisamment pure pour valoir un développement ultérieur et qui permet de comparer différents plasmides entre eux dans les conditions d'induction.

On va, dans les inductions décrites dans les exemples suivants, mesurer les cellules viables, le pourcentage de p+ et ensuite calculer le p-/ml/DO et le Fp-. Ces données seront présentées sous forme de tableaux.

EXEMPLE 8

Induction de l'hirudine

On présentera les résultats relatifs à l'induction de l'hirudine, en premier lieu, avec un vecteur contenant le gène de résistance à l'ampicilline, ensuite, avec le vecteur contenant aussi le gène dapD.

## 1) Stabilité des plasmides

### a) Induction de l'expression de l'hirudine dans un vecteur qui contient le gène de résistance à l'ampicilline (TGE900/pTG720) en LB à 37°C

Des résultats typiques pour TGE900/pTG720 sont repris dans le tableau 3.

TABLEAU 3

| Paramètre de stabilité des plasmides durant l'expression à 42°C de l'hirudine dans TGE900/pTG720 | | | | |
|---|---|---|---|---|
| Temps en heures | c/ml/DO | % p+ | p-/ml/DO | Fp- (%) |
| 0 h | $8,3.10^7$ | 100 | $< 1,7.10^6$ | $< 0,4$ |
| 1 h 30 | $2,4.10^8$ | 100 | $< 4,8.10^6$ | $< 1,2$ |
| 3 h | $2,1.10^8$ | 96 | $8,4.10^6$ | 2,1 |
| 4 h 30 | $4,7.10^7$ | 82 | $8,5.10^6$ | 2,1 |
| 5 h 30 | $1,8.10^7$ | 48 | $9,4.10^6$ | 2,35 |
| 7 h | $2,8.10^7$ | 20 | $2,2.10^7$ | 5,5 |

On voit qu'après 3 heures le nombre de cellules viables par unité de DO diminue en même temps que la proportion de p+. Après 5 h 30 le Fp- (% de p- par rapport à la totalité des cellules) est de l'ordre de 2,35 %. Après 7 heures, ce nombre aura doublé et il est vraisemblable que ceci est dû à la croissance des cellules p- uniquement.

### b) Induction de l'expression de l'hirudine dans un vecteur qui contient le gène dapD (TGE7615/pTG771) en LB à 37°C

Les résultats repris dans le tableau 4 sont comparables à ceux obtenus pour pTG720.

TABLEAU 4

| Paramètres de stabilité des plasmides durant l'expression à 42°C de l'hirudine dans TGE7615/pTG771 | | | | |
|---|---|---|---|---|
| Temps en heures | c/ml/DO | % p+ | p-/ml/DO | Fp- (%) |
| 0 h | $3,4.10^6$ | 99,8 | $7,7.10^5$ | 0,2 |
| 1 h 30 | $2,1.10^7$ | 99,2 | $1,7.10^5$ | 0,04 |
| 3 h | $9,7.10^6$ | 100 | $< 1,0.10^5$ | $< 0,025$ |
| 4 h | $9,3.10^6$ | 100 | $< 8,0.10^4$ | $< 0,005$ |
| 5 h | $7,5.10^6$ | 98 | $1,5.10^5$ | 0,04 |

On voit dans le tableau 4 que malgré une mortalité comparable à celle enregistrée pour TGE900/pTG771, la perte de plasmide (%p+) est faible (après 5 heures 98 % p+) tout comme la quantité absolue de cellules p- (p-/ml/DO). Après 5 heures le Fp- se quantifie à 0,04 %, ce qui est 50 fois moins que pour pTG720 au même moment. Ceci montre la meilleure stabilité du pTG771 par rapport au pTG720. Il faut rappeler que dans une expérience d'induction le pTG771 ne s etétramérise pas et que sa perte est supposée être inférieure à celle mesurée sur 170 générations à 30°C (voir tableau 2).

Les résultats montrent une plus grande stabilité du plasmide pTG771 que celle de pTG720.

## 2) Le contenu plasmidique

Les plasmides pTG720 et pTG771 ont été isolés à différents temps durant l'induction. De manière générale, on observe au début de l'induction, en phase exponentielle, un contenu plasmidique par cellule faible et qui s'accroît de manière sensible au cours du temps. Durant cette même période, il y a production d'hirudine.

Il est intéressant de noter que cette augmentation du contenu plasmidique se produit dans une population où plus de 95 % des cellules sont mortes.

3) L'activité induite

Les activités d'hirudine produites à partir des pTG720 ou pTG771 ne sont pas significativement différentes. Les valeurs (en unités antithrombine, UAT) sont de 2720 UAT/l/DO pour pTG720 et de 2380 UAT/l/DO pour pTG771, après 5 heures d'induction.

En conclusion, on peut dire que le pTG771 montre une stabilité accrue par rapport au pTG720, tout en gardant la même capacité de production et les mêmes propriétés d'augmentation de son nombre de copies en fin de phase exponentielle que le pTG720.

EXEMPLE 9

Induction de l'interféron gamma

Les données concernant l'induction de l'interféron gamma sont présentées de la même manière que celles de l'hirudine.

1) Stabilité des plasmides

a) Induction de l'expression de l'interféron gamma dans un vecteur contenant le gène de résistance à l'ampicilline (TGE900/pTG40) en LB à 42°C

Les résultats repris dans le tableau 5 montrent que la perte des plasmides est comparable à celle du pTG720 et qu'après 5 heures le Fp-s'élève à 6 %. Toutefois, à la différence de pTG720, la viabilité se perd plus vite et après 3 heures déjà un nombre minimal de cellules viables est atteint (à comparer avec les 5 heures observées pour le pTG720) ; de plus, les cellules p- présentes dans la culture restent viables et se divisent déjà significativement après 3 heures et contribuent à ce Fp- de 6 % après 5 heures.

TABLEAU 5

| Paramètres de stabilité des plasmides durant l'expression à 42°C de l'interféron dans TGE900/pTG40 | | | | |
|---|---|---|---|---|
| Temps en heures | c/ml/DO | % p + (Nb de colonies testées) | p-/ml/DO | Fp- (%) |
| 0 h | | | | |
| 1 h 30 | $1,8.10^6$ | 100 (202/202) | $< 10^6$ | < 1 |
| 3 h | $6,6.10^6$ | 10 (2/20) | $6,0.10^6$ | 1,5 |
| 5 h | $2,5.10^7$ | 2,7 (10/366) | $2,4.10^7$ | 6 |
| 6 h | $4,3.10^7$ | 1,7 (11/645) | $7,3.10^7$ | 18 |
| 8 h | $1,3.10^8$ | 0,9 (4/465) | $1,3.10^8$ | 32 |

b) Induction de l'expression de l'interféron dans un vecteur contenant le gène dapD (TGE7615/pTG7671) en LB à 42°C

Dans le tablerau 6, figurent les données relatives à une induction de TGE7615/pTG7671 à 42°C en LB. On remarque que la mortalité maximale n'est atteinte qu'après 5 heures et qu'à ce moment, bien que le %p + soit comparable à celui du pTG40 (10 %), le nombre des cellules p- (p-/ml/DO) est 5 fois inférieur à celui que l'on obtient après 3 heures pour le pTG40. En fait, après 5 heures le Fp- est de 0,3 % alors que le Fp- pour pTG40 est alors déjà de 6 %. Il y a donc une différence d'un facteur 20, qui est un reflet de la stabilité accrue du pTG7671, observation qui confirme les données de stabilité à 30°C (voir tableau 1).

TABLEAU 6

| Paramètres de stabilité des plasmides durant l'expression à 42°C de l'interféron dans TGE7615/pTG7671 | | | | |
|---|---|---|---|---|
| Temps en heures | c/ml/DO | % p+ (Nb de colonies testées) | p-/ml/DO | Fp- (%) |
| 0 h | $1,7.10^8$ | 100 (50/50) | $< 3,8.10^6$ | < 1 |
| 1 h 30 | $1,35.10^8$ | 100 (50/50) | $< 2,7.10^6$ | < 1,5 |
| 3 h | $4,2.10^7$ | 100 (50/50) | $< 8,4.10^5$ | < 0,2 |
| 5h | $1,3.10^6$ | 12 (6/50) | $1,1.10^6$ | 0,3 |
| 6 h | $2,2.10^6$ | 5 (5/100) | $2,1.10^6$ | 0.5 |

En conditions d'induction de TGE7615/pTG7671 à 42°C, la stabilité du plasmide est pratiquement identique en milieux sélectif et non-sélectif, la stabilité du pTG7671 étant déjà très bonne en absence de sélection. La perte du plasmide est donc réduite à un taux très satisfaisant et compatible avec une production industrielle.

2) Le contenu plasmidique

L'analyse sur gel d'agarose du contenu plasmidique montre que les souches transformées par les plasmides pTG40 et pTG7671 contiennent des quantités équivalentes de matériel et que ce contenu plasmidique par cellule augmente au cours du temps pendant l'induction. pTG40 est présent sous la forme dimère (comme montré sur le gel d'agarose).

3) Sélection des cellules p+ contenant le gène dapD

Afin de mettre en évidence le pouvoir sélectif du système dapD en tenant compte de la croissance des cellules p-, qui ne seront plus générées dès que la majeure partie des cellules auront perdu leur viabilité, on effectue une induction pendant laquelle la culture est diluée d'un facteur 5 à trois reprises espacées de 2 heures, après quoi on laisse cette culture atteindre la phase stationnaire. Les résultats pour l'induction à 42°C de TGE7615/pTG7671 sont présentés dans le tableau 7 pour une croissance en milieu sélectif LB et dans le tableau 8 pour une croissance en milieu non-sélectif LB + DAP.

TABLEAU 7

| Paramètres de stabilité des plasmides durant l'expression à 42°C de l'interféron dans TGE7615/pTG7671 en milieu sélectif LB | | | | |
|---|---|---|---|---|
| Temps en heures | c/ml/DO | % p+ (Nb de colonies testées) | p-/ml/DO | Fp- (%) |
| 0 h | $3,8.10^7$ | 100 (50/50) | $< 6.10^5$ | < 0,2 |
| 2 h | $8,3.10^7$ | 97 (74/76) | $1,4.10^6$ | 0,4 |
| 4 h | $< 10^6$ | | | |
| 6 h | $< 10^6$ | | | |
| 9 h | $3,7.10^6$ | 100 (60/60) | $< 3,7.10^4$ | < 0,01 |

TABLEAU 8

| Paramètres de stabilité des plasmides durant l'expression à 42 ° C de l'interféron dans TGE7615/pTG7671 en milieu LB + DAP | | | | |
|---|---|---|---|---|
| Temps en heures | c/ml/DO | % P + (Nb de colonies testées) | p-/ml/DO | Fp- (%) |
| 0 h | $5,5.10^7$ | 100 (50/50) | $< 1,2.10^6$ | < 0,3 |
| 2 h | $9,9.10^7$ | 99 (74/75) | $1,3.10^6$ | 0,3 |
| 4 h | $< 10^6$ | | | |
| 6 h | $< 10^6$ | | | |
| 9 h | $1,7.10^7$ | 5 (5/100) | $1,6.10^7$ | 4 |

On remarque dans le tableau 8 qu'au bout de 9 heures d'induction les cellules viables contiennent toutes du plasmide, en milieu sélectif LB (la présence du plasmide est confirmée par minipréparationà partir de 30 colonies sur un total de 60 colonies positives sur LB et identification du plasmide par électrophorèse sur gel d'agarose). En milieu non-sélectif, par contre, la viabilité est 5 fois plus élevée, mais la culture ne contient plus que 5 % de cellules ayant retenu le plasmide. Cette viabilité plus élevée en LB + DAP qu'en LB montre la croissance des cellules p- en milieu aditionné de DAP et a fortiori la mortalité des cellules p- dans le milieu sélectif. Donc le milieu LB permet une contre-sélection efficace des cellules p-.

4) Production de l'interféron

Le pTG7671 garde la même capacité de production de l'interféron gamma que le plasmide originel pTG40.

Exemple 10 Délétion du gène dapD du chromosome de la bactérie.

**Sous clonage du gène dapD sur 2 fragments PstI**

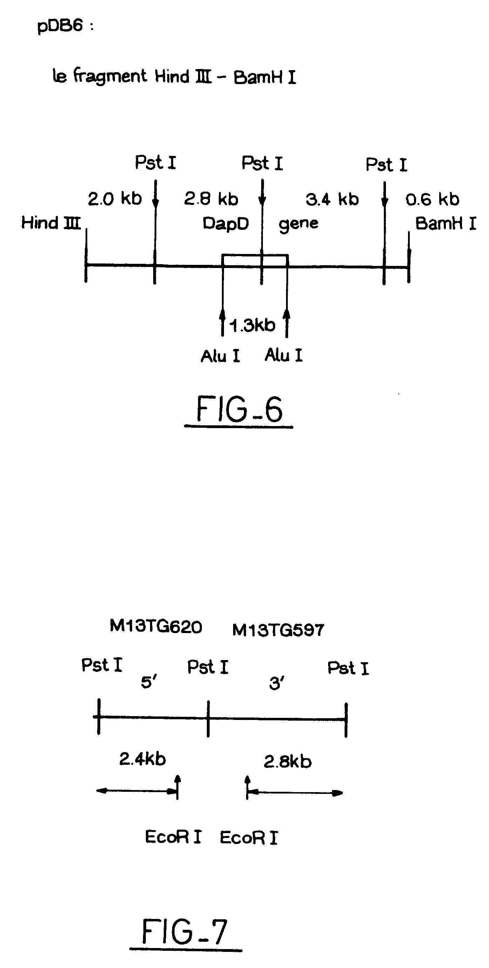

Le plasmide pDB6 et la M13mp8 sont coupés par PstI et ligués. On crible les M13 contenant l'extrémité 5' et l'extrémité 3' du gène par des oligonuctéotides spécifiques de ces régions, respectivement TG596 (GCGCTTAATAACGAGTTG) et TG598 (TGTGCATACTTTAGTC). Les candidats SB96 et SB98 sont retenus et l'insertion des fragments voulus confirmée par séquençage. (voir schéma de la figure 6).

**Introduction d'un site EcoRI dans les extrémités 5' et 3' du gène dapD**

Des sites EcoRI sont introduits dans SB96 et SB98 par mutation ponctuelle :
- dans SB98 avec l'oligonucléotide TG597 :
      GTACGCAGGAATTCCTTAATGCCG
   qui s'apparie avec la région 3' de la fin du gène, mais avant un terminateur de transcription supposé.
- dans SB96 avec l'oligonucléotide TG620 :
      AGAGGCCCGAATTCCAAACG
   qui s'apparie avec la région 5' en amont du promoteur supposé du gène dapD.
Les transformants sont analysés avec les mêmes sondes qui ont servi à introduire les sites EcoRI. La présence de ce site EcoRI est confirmée en minipréparation d'ADN et ensuite par séquençage des candidats M13 retenus (voir schéma figure 7 ).

**Construction d'un vecteur de délétion**

Le gène de résistance à la kanamycine du plasmide pUC-4K (vendu par Pharmacia) est récupéré sous forme de fragment EcoRI.
Les M13TG597 et 620 sont coupés par EcoRI et PstI ce qui libère respectivement l'extrémité 3' du gène dapD (sans le terminateur supposé) et l'extrémité 5' du gène (avec son promoteur supposé).
Le vecteur de clonage pTG192 (décrit dans l'exemple 2a) est coupé par PstI.
L'ensemble de ces fragments est ligué. Après transformation de cellules 5K et étalement sur LB + ampicilline 0.1 $\mu$g/ml + kanamycine 0.02 $\mu$g/ml, on crible les colonies par l'oligonucléotide TG596.

Une construction a été sélectionnée : pTG47. Sa structure est analysée par minipréparation d'ADN et l'orientation du gène de résistance à la kanamycine et déterminée par digestion du plasmide par HindIII, ce qui libère 2 bandes de tailles 5.3 et 4.0 kb. L'orientation du gène de résistance à la kanamycine dans pTG47 est la même que celle du gène dapD dans pDB6 (dans l'autre orientation on aurait obtenu des bandes de tailles 4.9 et 4.4 kb).

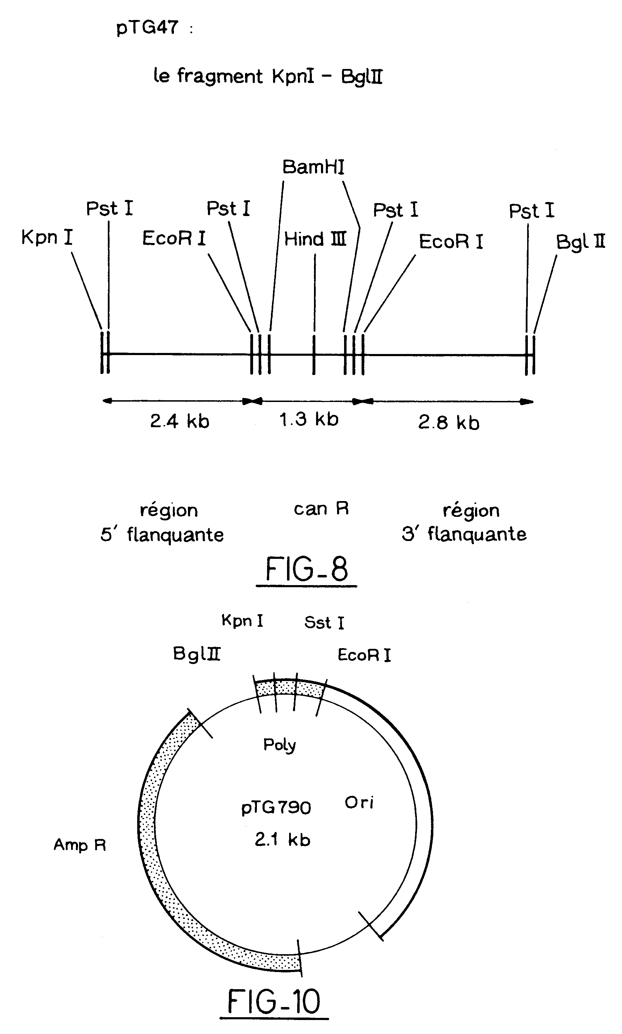

Le schéma du pTG47 est représenté dans la figure 8.

## Délétion du gène dapD du chromosome

Délétion du gène dapD du chromosome d'une souche intermédiaire

Des cellules RH5345, dont la compétence s'élève à 2.5 $10^{-7}$ transformants/µg d'ADN de pTG47, sont transformées par le plasmide pTG47 préalablement coupé par KnpI et BgIII (voir figure 8).

Cette digestion libère un fragment qui contient les régions flanquantes du gène dapD, le gène lui-même étant remplacé par le gène de résistance à la kanamycine.

Après étalement sur LB + DAP + kanamycine 0.01 µg/ml on sélectionne 9 candidats : TGE721 à TGE729 dont on confirme le phénotype dap$^-$, kan$^R$.

L'absence du gène dapD est confirmée après transformation de ces souches par le pTG764 par leur capacité de se multiplier en milieu LB.

Délétion du gène dapD de la souche TGE901 et N5969

La délétion dapD de le souche TGE721 est ensuite transduite dans les souches TGE901 et N5969 par le phage transducteur Plvir/TGE721 et les recombinants dap$^-$ sélectionnés par leur résistance à 0.01 µg/ml de kanamycine.

Les candidats retenus sont respectivement TGE7213, 7214 et TGE7303. Les exigences en ile, val, his de la souche mère, TGE901, en plus du caractère dap$^-$ kan$^R$ ont été confirmés sur les milieux appropriés, pour un candidat : TGE7214.

Exemple 11 Confirmation de la délétion du gène dap dans différentes souches par "blot" chromosomique.

Les différentes souches suivantes ont été analysées :
.   les souches parentales TGE901 et RH5345, dapD$^+$
.   les souches délétées TGE7213 et 7214
.   la souche parentale RL58 qui a été employée pour introduire la mutation dapD$^-$ dans TGE901, et le mutant dapD$^-$, TGE7615, qui en dérive
.   une souche GC4540 qui est résistante à la kanamycine par intégration du Tn5 alors que dans les souches TG le gène de résistance à la kanamycine dérive du Tn903 (les 2 gènes ne devraient pas donner d'hybridation croisée par manque d'homologie - Beck et al. 1982)

Le choix des enzymes de restriction est basé sur la séquence du pDB6 ; une coupure par :
-   BamHI et HindIII libère un fragment chromosomique de 9 kb contenant le gène dapD et ses régions flanquantes, dans le cas des souches sauvages ; dans le cas des souches délétées, le gène de résistance devra être libéré par la digestion par BamHI et coupé en 2 fragments par digestion par HindIII.
-   PstI libère 2 fragments contenant chacun une partie du gène dapD (2.8 kb de la région 5' et 3.4 kb de la région 3') pour les souches sauvages ; dans le cas des souches délétées la digestion par PstI libère le gène de résistance à la kanamycine et les 2 régions flanquantes (du côté 5' un fragment de 2.4 kb et du côté 3' un fragment de 2.8 kb) (voir figures 6 et 8).

Les sondes ont été choisies pour allumer le gène dap ou ses régions flanquantes ou le gène kan$^R$ :
-   pour sonder le gène de résistance à la kanamycine on isole un fragment EcoRI du pTG47 qui ne contient que ce gène de résistance (figure 8).
-   pour sonder le gène dapD on utilise un fragment EcoRI de 2.4 kb du M13TG620 ne contenant que la région 5' du gène dapD et qui devrait être entièrement délété dans TGE721, 7213, 7214 (figure 7).
-   pour sonder le gène DapD chromosomique et ses régions flanquantes, on utilise un fragment KpnI-BgIII de pTG47 qui contient le gène de résistance à la kanamycine et les 2 régions (le côté 5' et le 3') du chromosome qui flanquent le gène dapD (figure 8).

On s'attend donc à allumer,

- dans les souches sauvages, une bande de 2.8 kb correspondant à la région 5' du gène dapD et une bande de 3.4 kb correspondant à la région 3' de ce gène (figure 6).
- dans les souches délétées, le gène de résistance à la kanamycine, la région 5' flanquante du gène dapD qui subsiste (2.4 kb) et la région 3' flanquante du gène dapD qui subsiste (2.8 kb) (figure 8).

**Démonstration de l'incorporation du gène de résistance à la kanamycine**

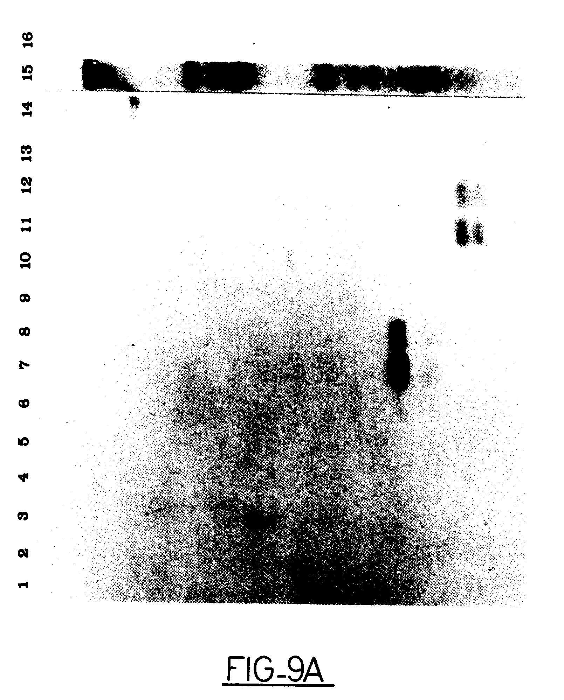

On a comparé les ADNs chromosomiques de GC4540, TGE7213, TGE7214 et TGE901, coupés par PstI ou BamHI et HindIII et sondé par le fragment EcoRI du pTG47. PstI libère la bande de 1.3 kb ; une restriction BamHI et HindIII donne 2 fragments de 0.7 et 0.6 kb pour les souches délétées uniquement. Aucune bande ne s'allume dans TGE901 ni dans GC4540 (figure 9A).

Ces résultats prouvent que le gène de résistance à la kanamycine est incorporé dans le chromosome des souches délétées et que ce gène provient du pUC-4K.

**Démonstration de la délétion du gène dapD du chromosome**

Les ADNs chromosomiques de GC4540, TGE7213, 7214 et TGE901 sont comparés avec comme contrôles M13TG620, M13TG597, le fragment BamHI-HindIII de pDB6 et le même fragment coupé par PstI (figure 6).

Les ADNs chromosomiques sont coupés par PstI ou BamHI et HindIII.

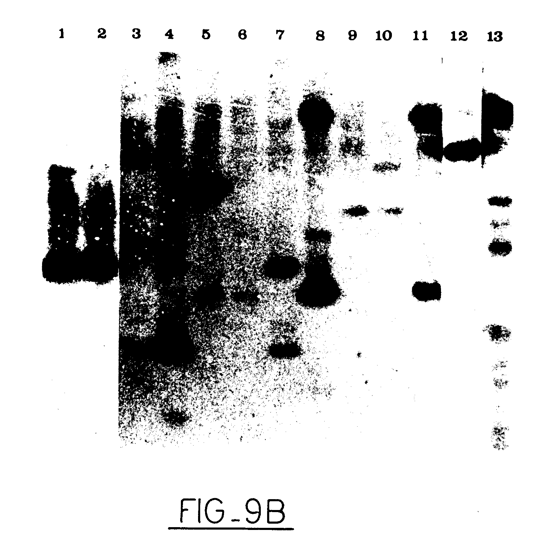

On isole M13TG620 coupé par EcoRI de la bande contenant spécifiquement le côté 5' du gène dapD pour l'utiliser comme sonde (figure 9B).

Après digestion par PstI on voit que dans les souches sauvages on allume une bande de 2.8 kb correspondant à la région 5' du gène dapD, mais aussi une bande inattendue de 1.7 kb.

Après digestion par BamHI et HindIII on allume une bande d'environ 12 kb qui est plus grande que la bande (de 9 kb) en provenance de pDB6. En plus une bande supplémentaire de 2.5 kb est aussi présente dans les souches sauvages (figure 9B).

Aucune homologie significative n'est apparente pour les souches délétées, dans aucun fragment de digestion.

Ces observations montrent que :
- une sonde qui couvre la partie 5' du gène dapD n'allume aucune bande dans le chromosome des souches TGE7213, 7214 ce qui démontre la délétion du côté 5' de ce gène.
- comme on allume dans les souches sauvages, en plus de la bande attendue, une deuxième bande, spécifique du gène dapD, il semble que ce gène soit dupliqué dans ces souches.

Comme une duplication du gène dapD était inattendue, nous avons confirmé cette duplication dans certaines souches sauvages, et confirmé la délétion du gène dap du côté 3' en plus du côté 5'.

Les mêmes ADNs chromosomiques et contrôles sont utilisés après digestion avec PstI (figure 8).

La sonde est le pTG47 coupé par KpnI et BglII. Cette sonde devrait allumer, en plus des bandes que l'on a prédites plus haut, au moins une bande de 1.7 kb. En effet le pTG47 contient 300 pb et 100 pb homologues au gène dapD, du côté 5' et du côté 3' respectivement, et qui devraient s'allumer dans les souches délétées.

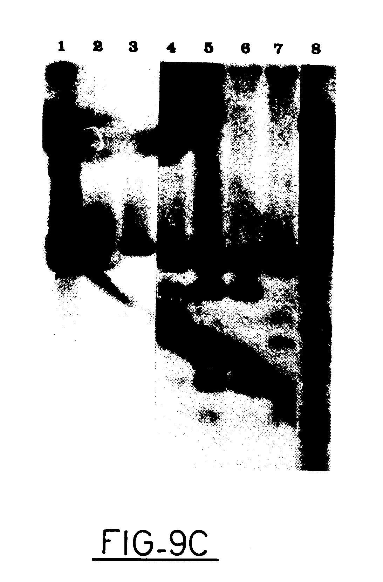

La figure 9C montre que :
- on allume des bandes de 2.8 et 2.4 kb dans les souches délétées et des bandes de 3.4 et 2.8 kb dans les souches sauvages ; en plus on allume une bande de 1.3 kb correspondant au gène de résistance à la kanamycine. Ceci prouve la délétion dans les souches TGE7213 et 7214.
- on allume aussi deux bandes moins intenses, dans les souches sauvages uniquement, dues au gène dapD dupliqué. Comme on sait que la bande de 1.7 kb s'allume avec la partie 5', la bande de 2.1 kb doit provenir du côté 3'. Ceci prouve que ces souches contiennent effectivement une duplication qui a disparu dans les souches délétées.

Nous avons comparé l'ADN chromosomique coupé par PstI des souches RH5345 et RL58 ainsi que la mutant dapD⁻ obtenu par conjugaison du RL58 avec TGE901. Ces ADNs sont sondés par le fragment KpnI, BglII isolé de pTG47 qui contient le gène de résistance à la kanamycine (qui ne devrait rien allumer) et les régions flanquantes du gène dapD.

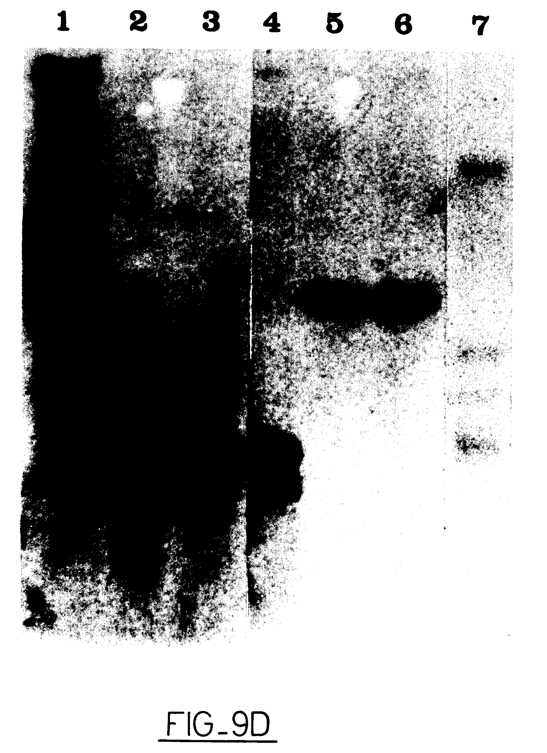

La figure 9D montre que dans RH5345 on n'allume que les bandes 3.4 et 2.8 kb et pas les bandes dues à la duplication du gène présentes dans GC4540 ou TGE901. De plus on ne remarqua qu'une bande de 7 kb pour RL58 et TGE7615, ce qui indique une perte d'un site PstI ; ceci prouve que ces 2 mutants sont identiques et touchés au moins dans le site PstI du gène dapD.

En conclusion, ces expériences montrent que :

- les souches TGE7213, 7214 sont délétées pour le gène dapD et qu'elles contiennent le gène de résistance à la kanamycine.
- la mutation dapD⁻ de RL58 et TGE7615 se trouve au moins au niveau du site PstI du gène dapD.
- certaines souches de E. coli présentent une duplication du gène dapD et cette duplication n'est pas présente dans les souches délétées.

Comme par transduction on réussit à déléter les 2 gènes dapD de la souche réceptrice, on peut conclure que le gène dupliqué doit se trouver proche (à moins de 2' sur la carte chromosomique de E. coli) du premier gène dapD.

Exemple 12 Clonage du gène cer.

Le gène cer est récupéré à partir du plasmide ColE1, dont la séquence a été publiée par Chan et al. (1985), sous forme d'un fragment HaeII de 1,85 kb.

Le fragment HaeII est ensuite coupé par HpaII, traité à la Klenow et une bande de 0.4 kb est récupérée. Le M13mp130 est coupé par EcoRV et traité à la phosphatase. On ligue le fragment de 0.4 kb du ColE1 dans le M13mp130 et on l'introduit dans la souche JM103. La présence du fragment cer de ColE1 a été confirmée par séquençage de la bande de 0.4 kb libérée par coupure SmaI et HindIII.

Le gène cer inséré dans le polylinker du M13mp131 est ensuite isolé après digestion par SmaI et HindIII et ligué dans le vecteur pTG720 (portant le gène hirudine, figure 2) coupé par BglII et traité à la Klenow. Le plasmide résultant est le pTG720cer.

Exemple 13 Construction de vecteurs de clonage contenant le gène cer et le gène dapD.

**Inversion du sens du polylinker de M13mp131 dans un vecteur codant pour la résistance à l'ampicilline**

Le pTG192 (figure 1) est coupé par EcoRI et BglII pour libérer le polylinker de M13mp131 et est raccourci par digestion HaeIII. On utilise un plasmide portant le gène de la résistance à l'ampicilline, par exemple le pTG730 (vecteur d'expression de l'hirudine décrit dans le brevet français 86.16723) ; ce plasmide est coupé par BglII et EcoRI et ligué au fragment EcoRI-BglII du pTG192. De cette manière on perd le bloc d'expression comprenant le $P_L$ et le gène de structure de l'hirudine du pTG730 que l'on remplace par le polylinker de M13mp131. On appelle ce nouveau plasmide pTG790 (figure 10).

**Introduction du fragment cer dans un vecteur de clonage**

Le pTG790 est coupé par SstI et KpnI et traité à la phosphatase. Le fragment résultant de cette digestion est ligué au pTG720cer, coupé par SstI et KpnI (ce qui libère le fragment cer) et raccourci par digestion BglII. Le vecteur résultant, pTG792, contient le fragment cer (figure 11).

**Introduction du gène dapD dans un vecteur contenant le fragment cer**

Le pTG792 est coupé par EcoRI, traité à la Klenow et à la phosphatase. Le fragment résultant est ligué avec le fragment AluI de 1.3 kb issu de pDB6 qui contient le gène dapD (figure 6). Il en résulte 2 plasmides pTG7922 et pTG7923 qui ne diffèrent que par l'orientation du gène dapD situé entre 2 sites EcoRI. Pour le pTG7922 les promoteurs des 3 gènes, origine de réplication, résistance à l'ampicilline et dapD sont orientés de la même manière (figure 12).

**Délétion du gène de résistance à l'ampicilline**

Les constructions suivantes ont un but multiple :
- déléter le gène de résistance à l'ampicilline des vecteurs contenant le gène dapD ;
- obtenir un vecteur de clonage dapD contenant le gène cer ;
- obtenir un vecteur de clonage dapD qui contient le polylinker de M13mp131 (moins le site EcoRV, utilisé pour le clonage du cer) ;
- obtenir un vecteur dapD avec un seul site EcoRI.

On récupère un fragment PstI du pTG7922 et du pTG7923 contenant respectivement la partie 3' et 5' du gène dapD ainsi que le gène cer pour l'introduire dans un vecteur dap contenant un fragment analogue mais pas de site EcoRI ou AvaI, ni le cer. Les vecteurs analogues sont respectivement le pTG767 et le pTG766 décrits précédemment.

Le pTG7922 et pTG7923 sont coupés par PstI, raccourcis par digestion BglII et ligués respectivement dans pTG767 et pTG766 coupés par PstI et traités à la phosphatase. Les vecteurs de clonage qui en résultent sont respectivement pTG769 et pTG768 (le pTG769 est représenté dans la figure 13).

Exemple 14 Application du modèle dap à la construction de vecteurs d'expression pour la catéchol 2,3 oxygénase ($C_{2,3}O$).

**Vecteur sans site BamHI en amont de la $C_{2,3}O$**

Le gène de structure de la $C_{2,3}O$ est récupéré à partir du pTG444 pour être introduit dans le vecteur dap pTG7671 (décrit précédemment).

Le pTG444, est identique au pTG445 décrit par Zukowski et al. (1984) à l'exception d'un site XmaIII non régénéré. Le pTG444 est coupé par BamHI et HindIII et ligué au pTG769 coupé par BamHI et HindIII et traité à la phosphatase. Le plasmide résultant, appelé pTG7401 contient le gène de structure de la $C_{2,3}O$.

Le pTG7671 contient 2 sites BglII : un site en amont du $P_L$ faisant partie du polylinker et un site en aval du $P_L$ situé dans le site d'attachement des ribosomes, en amont du gène de structure de l'interféron gamma. Le pTG7671 est coupé par BglII et raccourci par digestion KpnI. Le mélange résultant est ligué dans le pTG7401 coupé dans son polylinker par BglII et BamHI et traité à la phosphatase. Par cette ligation on reconstitue le site BglII mais le site BamHI ligué au BglII est perdu. Deux orientations du $P_L$ par rapport au gène de structure de la $C_{2,3}O$ sont possibles. Pour distinguer la construction où la $C_{2,3}O$ est sous le contrôle du $P_L$, on coupe par BamHI et BglII (en effet pour l'orientation voulue un site BamHI se trouve à proximité du site BglII et la digestion ne donnera pratiquement qu'une bande de 4.3 kb ; dans l'autre orientation la digestion libère une bande à 3.9 kb et une bande de 0.4 kb).

Le plasmide retenu, pTG7407, ayant la $C_{2,3}O$ sous contrôle du $P_L$, a une structure voisine du pTG7406 décrit plus loin (comparer à la figure 14) mais il a perdu le site BamHI en amont de la $C_{2,3}O$.

**Vecteur avec un site BamHI en amont de la $C_{2,3}O$**

Le pTG769 est coupé par BglII et BamHI, traité à la phosphatase et ligué avec un fragment BamHI-BglII d'un plasmide d'expression quelconque (pTG907) qui contient le $P_L$ et le gène N complet, de λ. Il en résulte une construction, le pTG7400, qui peut être identifiée par une coupure BamHI et BglII qui libère 2 bandes : à 2.6 kb et 1.3 kb. Cette construction contient le $P_L$ et le gène N complet.

Le pTG7400 est ensuite coupé par HpaI et on y insère un linker BamHI (vendu par BRL) CCGGATCCGG, phosphorylé et hybridé. Ceci donne le pTG7402 qui a perdu son site HpaI mais contient 2 sites BamHI.

Le pTG7402 est coupé par BamHI et religué pour donner le pTG7404. Par cette manipulation on a éliminé un site BamHI et tronqué le gène N.

**Introduction du gène de la $C_{2,3}O$ dans un vecteur dap-cer**

Le pTG7402 est coupé par BamHI et HindIII, traité à la phosphatase et le fragment BamHI-HindIII du pTG444 y est introduit pour donner le pTG7406 (voir figure 14). Il diffère du pTG7407 par le fait qu'on peut sortir le gène de la $C_{2,3}O$ par une coupure BamHI, HindIII pour récupérer le fragment introduit à partir de pTG444.

**Expression de la $C_{2,3}O$ dans les souches de E. coli dap$^-$ transformées par le plasmide pTG7407**

L'expression du gène $C_{2,3}O$ dans les bactéries TGE7213/pTG7407 a été mesurée à 30°C après 4 h et 7 h de culture et au cours de l'induction à 42°C après 4 h et 7 h.
Pour chaque détermination, un échantillon de la culture est récolté et centrifugé ; le culot est lavé et repris en tampon phosphate (comme décrit par Zukowski et al. 1983) puis traité aux ultra-sons pendant 3 fois 20 secondes.
Après centrifugation 10 min. à 10.000 g, on considère le culot comme la fraction insoluble (C) et le surnageant comme la fraction soluble (S).

Les protéines présentes dans chaque fraction sont analysées par électrophorèse sur gel de polyacrylamide-SDS. Les bandes sont révélées par coloration au bleu de Coomassie.
Les résultats sont présentés dans la figure 15. On remarque l'intensité de la bande de PM 35.000, en particulier après 7 h d'induction à 42°C.

Le "scanning" du gel donne environ 64 % et 75 % de $C_{2,3}O$ dans les fractions S et C respectivement.

Dans l'échantillon le plus riche (S, 7 h à 42°C), on a déterminé l'activité spécifique de la $C_{2,3}O$ (selon la méthode décrite par Zukowski et al. 1983), par addition de catéchol comme substrat. On mesure une activité spécifique de 28 à 35 U/mg.

L'activité spécifique d'une préparation d'enzyme pure étant de 280 U/mg, les extraits analysés contiennent environ 12 % de $C_{2,3}O$ active.

Exemple 15 Introduction du fragment cer dans un vecteur d'expression de l'interféron gamma.

Le pTG7671 décrit précédemment est coupé au niveau de son polylinker par SstI (identique à SacI) et KpnI puis traité à la phosphatase et ligué aux fragments de pTG720cer coupé par SstI et KpnI. Après transformation dans TGE7615 on retient un candidat, pTG7675, qui libère 2 fragments de 400 pb et de 3.4 kb après digestion par SstI et KpnI (figure 16).

Exemple 16 Mise en évidence de la stabilité des plasmides au cours de l'induction de l'expression de l'interféron gamma.

a) Induction de l'expression de l'interféron gamma en milieu LB à 42°C pour un vecteur portant le gène de résistance à l'ampicilline : TGE901/pTG40.

Les résultats sont repris dans le tableau 9. On mesure les cellules totales/ml/unité de DO afin de déterminer que la perte de la viabilité est un phénomène réel et non un changement de volume des cellules, par exemple. Ces données sont aussi reprises dans le tableau 9. Le $Fp^-$ a été défini dans l'exemple 7 et relie le nombre de cellules $p^-$ au nombre total de cellules présentes à un moment donné.

On remarquera que le $Fp^-$ après 7h30 d'induction atteint une valeur de près de 2 %. Il est donc moins élevé que dans l'expérience précédente (voir exemple 9), ce qu'on ne peut attribuer qu'à une différence dans la structure du plasmide pTG40 dans ces 2 expériences : en effet, quoique la quantité de plasmide soit équivalente à différents moments de l'induction, dans la première expérience le plasmide était sous forme dimérique alors que dans l'expérience décrite ici il est principalement sous forme monomérique (comme le montre une analyse sur gel). L'état du plasmide n'influence pas la production en interféron gamma mais illustre de manière concrète la perte de stabilité si la forme monomérique d'un plasmide n'est pas maintenue.

b) Induction de l'expression de l'interféron gamma dans une souche mutée pour le gène dap et transformée par un vecteur contenant le gène dapD et le gène cer, TGE7615/pTG7675

Les résultats sont repris dans le tableau 10. On mesure les cellules totales/ml/unité de DO ; il n'y a pas de différence notable avec TGE901/pTG40. On confirme donc une réelle perte de la viabilité : après 7h30 d'induction, seulement 0.02 % de la culture reste viable. Ceci est à comparer avec le TGE901/pTG40 où la valeur obtenue est de 2.4 %. Ceci se traduit au niveau du $Fp^-$ qui, dans le cas de TGE7615/pTG7675, est diminué d'un facteur 1000 par rapport au TGE901/pTG40. Pourtant il reste toujours quelques cellules $p^-$ qui apparaissent en fin d'induction. Le contenu plasmidique présente les mêmes caractéristiques qu'avant, c'est-à-dire augmentation du nombre de copies en fin de croissance mais les formes multimériques présentes en plus ou moins grand nombre avec les plasmides sans cer, sont quasi absentes ici. La production en interféron gamma est légèrement supérieure à celle obtenue avec le pTG40.

c) Induction de l'expression de l'interféron gamma dans une cellule hôte délétée pour le gène dapD et transformée par un vecteur contenant le gène dapD et le cer, TGE7213/pTG7675

Les résultats sont repris dans le tableau 11. Les conclusions sont identiques à celles tirées de la comparaison entre TGE901/pTG40 et TGE7615/pTG7675 : la mortalité atteint un facteur $3.5 \ 10^{-4}$, la quantité de cellules totales/ml/unité de DO ne change pas de manière significative durant l'induction. Par contre même après 7h30 d'induction il n'y a pas d'apparition de cellules $p^-$ (après 24h toute la culture est devenue $p^-$ pour TGE901/pTG40 et est restée 100 % $p^+$ pour TGE7213/pTG7675). Le contenu plasmidique est comparable à celui de TGE7615/ pTG7675 par l'absence de multimères. La production en interféron gamma est légèrement supérieure à celle obtenue avec TGE7615/pTG7675.

## Tableau 9

### Induction de TGE901/pTG40 à 42°C en milieu LB

| | D.O. 600 nm | c/ml/D.O. | c totales/ml/D.O. | Fp⁻ |
|---|---|---|---|---|
| 0 h 00 | 0.370 | $3.9 \ 10^8$ | | $< 1.4$ % |
| 1 h 00 | 1.29 | $3.8 \ 10^8$ | $6.5 \ 10^8$ | |
| 2 h 00 | 1.67 | $3.0 \ 10^7$ | $7.2 \ 10^8$ | 0.03 % |
| 3 h 00 | 2.13 | $2.7 \ 10^6$ | | 0.11 % |
| 4 h 00 | 2.42 | $2.8 \ 10^6$ | $6.7 \ 10^8$ | 0.23 % |
| 6 h 15 | 2.89 | $7.0 \ 10^6$ | $6.5 \ 10^8$ | 0.70 % |
| 7 h 30 | 3.08 | $9.5 \ 10^6$ | | 1.73 % |

NOTE : Fp⁻ est le pourcentage des p⁻ sur la totalité des cellules présentes à un moment donné.

EP 0 258 118 B1

EP 0 258 118 B1

## Tableau 10

### TGE7615/pTG7675 à 42°C en milieu LB

|  | D.O. 600 nm | c/ml/D.O. | c totales/ml/D.O. | Fp⁻ |
|---|---|---|---|---|
| 0 h 00 | 0.310 | $3.5 \ 10^8$ |  |  |
| 1 h 00 | 1.07 | $3.3 \ 10^8$ | $4.7 \ 10^8$ |  |
| 2 h 00 | 1.64 | $1.5 \ 10^8$ | $5.1 \ 10^8$ |  |
| 3 h 00 | 2.31 | $1.44 \ 10^7$ |  |  |
| 4 h 00 | 2.67 | $7.1 \ 10^6$ | $7.9 \ 10^8$ |  |
| 6 h 15 | 3.26 | $6.1 \ 10^4$ | $6.1 \ 10^8$ | 0.0017 % |
| 7 h 30 | 3.44 | $6.4 \ 10^4$ |  | 0.0018 % |

**Tableau 11**

Induction de TGE7214/pTG7675 à 42°C en milieu LB

| | D.O. 600 nm | c/ml/D.O. | c totales/ml/D.O. | Fp⁻ |
|---|---|---|---|---|
| 0 h 00 | 0.400 | $3.4 \ 10^8$ | | |
| 1 h 00 | 1.25 | $3.7 \ 10^8$ | $5.8 \ 10^8$ | $< 1.1$ * |
| 2 h 00 | 1.71 | $3.8 \ 10^7$ | $7.1 \ 10^8$ | |
| 3 h 00 | 2.45 | $3.0 \ 10^5$ | | |
| 4 h 00 | 3.32 | $6.6 \ 10^4$ | $12.8 \ 10^8$ | |
| 6 h 15 | 4.25 | $7.3 \ 10^4$ | $5.8 \ 10^8$ | $< 6 \ 10^{-4}$ * |
| 7 h 30 | 4.21 | $1.2 \ 10^5$ | | |

**Exemple 17** Application du modèle dap à la construction d'un vecteur d'expression pour l'alpha-1 antitrypsine.

Le fragment PstI contenant le bloc d'expression de l'alpha-1 antitrypsine c'est-à-dire le promoteur $P_L$ du phage lambda, le gène N tronqué, un site d'attachement des ribosomes et le gène de structure de l'alpha-1

antitrypsine (Arg$^{358}$) provenant du pTG2901 (dérivé tronqué du pTG983, décrit dans le brevet français 85.07393) est introduit dans le pTG792 (décrit plus haut) coupé par PstI et traité à la phosphatase.

Le vecteur d'expression résultant, pTG7913, est ensuite coupé par BglII et SstI et le bloc d'expression contenant l'alpha-1 antitrypsine et le gène cer est introduit dans le pTG767 coupé par BglII et SstI et traité à la phosphatase.

Le plasmide résultant, pTG7914, contient le gène dapD, le gène cer et le bloc d'expression de l'alpha-1 antitrypsine (Arg$^{358}$) (figure 17).

**Dépôt des souches représentatives de l'invention**

Les souches suivantes ont été déposées à la Collection Nationale de Cultures des Microorganismes (25 Rue du Dr. Roux, Paris) :
- TGE 7615/pTG7671 sous le n° I-586
- TGE 7615/pTG771 sous le n° I-585 le 25.07.86
- TGE7214, souche de coli délétée du gène dapD, sous le n° I-652 le 10.03.87
- TGE7303, souche de coli délétée du gène dapD, sous le n° I-653 le 10.03.87 (les 2 souches sont transformées par le plasmide pTC768 qui porte le gène dapD et cer).
- TGE7214/pTG7407, souche dapD$^{-}$ transformée par le plasmide d'expression de la C$_{2,3}$O, sous le n° I-655, le 10/03/87

REFERENCES

1. Jones I.M., Primrose S.B., Robinson A., Ellwood D.C. (1980) Mol. Gen. Genet. 180, 579-584.
2. Nilsson J., Skogman G. (1985) European patent application n° 84850313.2.
3. Skogman G., Nilsson J. (1984) Gene 31, 117-122.
4. Miwa K. Nakamori S., Sano K., Momose H. (1984) Agric. Biol. Chem. 48, 2233-2237.
5. Miwa K., Nakamori S., Sano K. Momose H., 1984) Gene 31, 275-277.
6. Hershberger C.L., Rosteck P.R. (1984) United States Patent n° 4 436 815.
7. Work E. (1950) Nature 165, 74-75.
8. Davis B.D., Dulbecco R., Eisen H.N., Ginsberg H.S., Wood W.B. (2nd Ed. 1973) Microbiology Ed. Harper International p. 72.
9. Dauce-Le Reverend B., Boitel M., Deschamps A.M., Lebeault J-M., Sano K., Takinami K., Patte J-C. (1982) European J. Appl. Microbiol. Biotechnol. 15, 227-231.
10. Richaud C., Richaud F., Martin C., Haziza C., Patte J-C. (1984) J. Biol. Chem. 259, 14824-14828.
11. Hanahan D. (1983) J. Mol. Biol. 166, 557-580.
12. Bollen A., Lathe R., Herzog A., Denicourt D., Lecocq J-P., Demarez L., Lavalle R. (1979) J. Mol. Biol. 132, 219-233.
13. Gottesman M.E., Adhya S., Das A. (1980) J. Mol. Biol. 140, 57-75.
14. Lusky M., Botchan M.(1981) Nature 293, 79-81.
15. Vieira J., Messing J. (1982) Gene 22, 259-268.
16. Lathe R., Kieny M.P., Skory S., Lecocq J-P. (1984) DNA 3, 173-182.
17. Kieny M-P., Lathe R., Lecocq J-P. (1983) Gene 26, 91-99.
18. Bendiak D.S., Friesen J.D. (1981) Mol. Gen. Genet. 181, 356-362.
19. Summers D.J., Sherratt D.J. (1984) Cell 36, 1097-1103.
20 Beck, E., Ludwig, G., Auerswald, E.A., Reiss, B. & Schaller, M. Gene 19, 327-336 (1982).
21 Bendiak, D.S. & Friesen, J.D. Mol. Gen. Genet. 181, 356-362 (1981).
22 Bollen, A., Lathe, R., Herzog, A., Denicourt, D., Lecocq, J.P., Desmarez, L. & Lavallé, R. J. Mol. Biol. 132, 219-233 (1979).
23 Bukhari, A.I. & Taylor, A.L. J. Bacteriol. 105, 844-854 (1971).
24 Chan, P.T., Ohmori, H., Tomizawa, J.I & Lebowitz, J. J. Biol. Chem. 260, 8925-8935 (1985).
25 D'Ari, R. & Huisman, O. J. Bacteriol. 156, 243-250 (1983).
26 Richaud, C., Richaud, F., Martin, C., Haziza, C. & Patte, J.C J. Biol. Chem. 259, 14824-14828, 1984.
27 Summers, D.K. & Spherratt, Cell 36, 1097-1103 (1984).
28 Zukovski, M.M., Gaffney, D.F., Speck, D., Kauffmann, M., Findeli, A., Wisecup, A. & Lecocq, J.P. Proc. Natl. Acad. Sci. USA 80, 1101-1105 (1983).
29 Zukowski, M.M., Speck, D., Kauffmann, M. & Lecocq, J.P. Genetics and Biotechnology of Bacilli 309-319 (1984).

**Revendications**
**Revendications pour les Etats contractants suivants : AT, BE, CH, LI, DE, FR, GB, IT, LU, NL, SE**

1. Souche bactérienne comportant une mutation du gène dapD et transformée par un vecteur plasmidique portant un gène dapD intact, ainsi qu'un gène codant pour une protéine d'intérêt, et les éléments assurant son expression dans la bactérie hôte.

2. Souche bactérienne selon la revendication 1, caractérisée en ce que la mutation chromosique dapD est une délétion d'une partie au moins du gène dapD et en ce que le vecteur plasmidique porte un gène dapD intact.

3. Souche bactérienne selon la revendication 2, caractérisée en ce que la délétion est une délétion totale du gène dapD.

4. Souche selon l'une des revendications 1 à 3, caractérisée en ce que le vecteur plasmidique porte une séquence qui le maintient à l'état de monomère.

5. Souche bactérienne selon la revendication 4, caractérisée en ce que ladite séquence est une séquence "cer".

6. Souche bactérienne selon l'une des revendications 1 à 5, caractérisée en ce que la bactérie hôte est une souche de E. Coli.

7. Souche selon l'une des revendications 5 ou 6, caractérisé en ce que la protéine d'intérêt industriel est choisie parmi l'hirudine, l'interféron gamma, la Catéchol$_{2,3}$Oxygénase et l'alpha-antitrypsine.

8. Procédé de préparation d'une protéine d'intérêt industriel, caractérisé en ce qu'on cultive une souche selon l'une des revendications 1 à 7 et on récupère ladite protéine d'intérêt.

9. Procédé de préparation de C$_{2,3}$O caractérisé en ce qu'on cultive sur un milieu complet une souche selon l'une des revendications 1 à 7, dans laquelle le gène codant pour l'expression d'une protéine d'intérêt industriel est le gène codant pour le C$_{2,3}$O.

10. Procédé de préparation de l'hirudine, caractérisé en ce qu'on cultive sur un milieu complet une souche selon l'une des revendications 1 à 7, dans laquelle le gène codant pour l'expression d'une protéine d'intérêt industriel est le gène codant pour une hirudine ou l'un de ses variants naturels ou artificiels.

11. Procédé de préparation de l'interféron gamma, caractérisé en ce qu'on cultive sur un milieu complet une souche selon l'une des revendications 1 à 7 dans laquelle le gène codant pour l'expression d'une protéine d'intérêt industriel est le gène codant pour l'interféron gamma.

**Revendications pour les Etats contractants suivants : ES, GR**

1. Souche bactérienne comportant une mutation du gène dapD et transformée par un vecteur plasmidique portant un gène dapD intact, ainsi qu'un gène codant pour une protéine d'intérêt, et les éléments assurant son expression dans la bactérie hôte.

2. Souche bactérienne selon la revendication 1, caractérisée en ce que la mutation chromosique dapD est une délétion d'une partie au moins du gène dapD et en ce que le vecteur plasmidique porte un gène dapD intact.

3. Souche bactérienne selon la revendication 2, caractérisée en ce que la délétion est une délétion totale du gène dapD.

4. Souche selon l'une des revendications 1 à 3, caractérisée en ce que le vecteur plasmidique porte une séquence qui le maintient à l'état de monomère.

EP 0 258 118 B1

5. Souche bactérienne selon la revendication 4, caractérisée en ce que ladite séquence est une séquence "cer".

6. Souche bactérienne selon l'une des revendications 1 à 5, caractérisée en ce que la bactérie hôte est une souche de E. Coli.

7. Souche selon l'une des revendications 5 ou 6, caractérisé en ce que la protéine d'intérêt industriel est choisie parmi l'hirudine, l'interféron gamma, la Catéchol$_{2,3}$ Oxygénase et l'alpha-antitrypsine.

8. Procédé de préparation d'une souche selon l'une des revendications 1 à 7 dans lequel on transforme une souche comportant une mutation du gène dapD par un vecteur plasmidique portant un gène dapD intact, ainsi qu'un gène codant pour une protéine d'intérêt, et les éléments assurant son expression dans la bactérie hôte.

9. Procédé de préparation d'une protéine d'intérêt industriel, caractérisé en ce qu'on cultive une souche selon l'une des revendications 1 à 7 et on récupère ladite protéine d'intérêt.

10. Procédé de préparation de C$_{2,3}$O caractérisé en ce qu'on cultive sur un milieu complet une souche selon l'une des revendications 1 à 7, dans laquelle le gène codant pour l'expression d'une protéine d'intérêt industriel est le gène codant pour le C$_{2,3}$O.

11. Procédé de préparation de l'hirudine, caractérisé en ce qu'on cultive sur un milieu complet une souche selon l'une des revendications 1 à 7, dans laquelle le gène codant pour l'expression d'une protéine d'intérêt industriel est le gène codant pour une hirudine ou l'un de ses variants naturels ou artificiels.

12. Procédé de préparation de l'interféron gamma, caractérisé en ce qu'on cultive sur un milieu complet une souche selon l'une des revendications 1 à 7 dans laquelle le gène codant pour l'expression d'une protéine d'intérêt industriel est le gène codant pour l'interféron gamma.

## Claims
## Claims for the following Contracting States : AT, BE, CH, LI, DE, FR, GB, IT, LU, NL, SE

1. Bacterial strain containing a mutation of the dapD gene and transformed by a plasmid vector carrying an intact dapD gene, as well as a gene coding for a protein of value, and the elements ensuring its expression in the host bacterium.

2. Bacterial strain according to Claim 1, characterized in that the dapD chromosomal mutation is a deletion of at least a portion of the dapD gene and in that the plasmid vector carries an intact dapD gene.

3. Bacterial strain according to Claim 2, characterized in that the deletion is a total deletion of the dapD gene.

4. Bacterial strain according to one of Claims 1 to 3, characterized in that the plasmid vector carries a sequence which maintains it in the monomeric state.

5. Bacterial strain according to Claim 4, characterized in that the said sequence is a "cer" sequence.

6. Bacterial strain according to one of Claims 1 to 5, characterized in that the host bacterium is a strain of E. coli [sic].

7. Strain according to either of Claims 5 and 6, characterized in that the protein of industrial value is chosen from amongst hirudin, interferon-gamma, catechol$_{2,3}$ Oxygenase and alpha-antitrypsin.

8. Method for the preparation of a protein of industrial value, characterized in that a strain according to one of Claims 1 to 7 is cultured and the said protein of value is recovered.

24

9. Method for the preparation of $C_{2,3}O$, characterized in that a strain according to one of Claims 1 to 7 in which the gene coding for the expression of a protein of industrial value is the gene coding for $C_{2,3}O$ is cultured in a complete medium.

10. Method for the preparation of hirudin, characterized in that a strain according to one of Claims 1 to 7 in which the gene coding for the expression of a protein of industrial value is the gene coding for a hirudin or one of its natural or artificial variants is cultured in a complete medium.

11. Method for the preparation of interferon-gamma, characterized in that a strain according to one of Claims 1 to 7 in which the gene coding for the expression of a protein of industrial value is the gene coding for interferon-gamma is cultured in a complete medium.

**Claims for the following Contracting States : ES, GR**

1. Bacterial strain containing a mutation of the dapD gene and transformed by a plasmid vector carrying an intact dapD gene, as well as a gene coding for a protein of value, and the elements ensuring its expression in the host bacterium.

2. Bacterial strain according to Claim 1, characterized in that the dapD chromosomal mutation is a deletion of at least a portion of the dapD gene and in that the plasmid vector carries an intact dapD gene.

3. Bacterial strain according to Claim 2, characterized in that the deletion is a total deletion of the dapD gene.

4. Bacterial strain according to one of Claims 1 to 3, characterized in that the plasmid vector carries a sequence which maintains it in the monomeric state.

5. Bacterial strain according to Claim 4, characterized in that the said sequence is a "cer" sequence.

6. Bacterial strain according to one of Claims 1 to 5, characterized in that the host bacterium is a strain of E. coli [sic].

7. Strain according to either of Claims 5 and 6, characterized in that the protein of industrial value is chosen from amongst hirudin, interferon-gamma, catechol$_{2,3}$Oxygenase and alpha-antitrypsin.

8. Method for the preparation of a strain according to one of Claims 1 to 7, in which a strain containing a mutation of the dapD gene is transformed by a plasmid vector carrying an intact dapD gene, as well as a gene coding for a protein of value, and the elements ensuring its expression in the host bacterium.

9. Method for the preparation of a protein of industrial value, characterized in that a strain according to one of Claims 1 to 7 is cultured and the said protein of value is recovered.

10. Method for the preparation of $C_{2,3}O$, characterized in that a strain according to one of Claims 1 to 7 in which the gene coding for the expression of a protein of industrial value is the gene coding for $C_{2,3}O$ is cultured in a complete medium.

11. Method for the preparation of hirudin, characterized in that a strain according to one of Claims 1 to 7 in which the gene coding for the expression of a protein of industrial value is the gene coding for a hirudin or one of its natural or artificial variants is cultured in a complete medium.

12. Method for the preparation of interferon-gamma, characterized in that a strain according to one of Claims 1 to 7 in which the gene coding for the expression of a protein of industrial value is the gene coding for interferon-gamma is cultured in a complete medium.

**Patentansprüche**
**Patentansprüche für folgende Vertragsstaaten : AT, BE, CH, LI, DE, FR, GB, IT, LU, NL, SE**

1. Bakterienstamm, der eine Mutation des Gens dapD enthält und durch einen Plasmid-Vektor transformiert worden ist, der ein intaktes Gen dapD sowie ein Gen, das für ein interessierendes Protein codiert, und die Elemente, die seine Expression in dem Wirtsbakterium gewährleisten, trägt.

2. Bakterienstamm nach Anspruch 1, dadurch gekennzeichnet, daß die Chromosomen-Mutation dapD eine Deletion mindestens eines Teils des Gens dapD ist und daß der Plasmid-Vektor ein intaktes Gen dapD trägt.

3. Bakterienstamm nach Anspruch 2, dadurch gekennzeichnet, daß die Deletion eine Gesamtdeletion des Gens dapD ist.

4. Bakterienstamm nach einem der Ansprüche 1 bis 3, dadurch gekennzeichnet, daß der Plasmid-Vektor eine Sequenz trägt, die ihn im monomeren Zustand hält.

5. Bakterienstamm nach Anspruch 4, dadurch gekennzeichnet, daß die genannte Sequenz eine Sequenz "cer" ist.

6. Bakterienstamm nach einem der Ansprüche 1 bis 5, dadurch gekennzeichnet, daß das Wirts-Bakterium ein Stamm von E. coli ist.

7. Bakterienstamm nach einem der Ansprüche 5 oder 6, dadurch gekennzeichnet, daß das Protein von industriellem Interesse ausgewählt wird aus Hirudin, Gamma-Interferon, Brenzkatechin-2,3-Oxygenase und $\alpha$-Antitrypsin.

8. Verfahren zur Herstellung eines Proteins von industriellem Interesse, dadurch gekennzeichnet, daß man einen Stamm nach einem der Ansprüche 1 bis 7 kultiviert und das genannte interessierende Protein abtrennt (gewinnt).

9. Verfahren zur Herstellung von $C_{2,3}O$, dadurch gekennzeichnet, daß man auf einem vollständigen Medium einen Stamm nach einem der Ansprüche 1 bis 7 kultiviert, in dem das Gen, das für die Expression eines Proteins von industriellem Interesse codiert, das Gen ist, das für $C_{2,3}O$ codiert.

10. Verfahren zur Herstellung von Hirudin, dadurch gekennzeichnet, daß man auf einem vollständigen Medium einen Stamm nach einem der Ansprüche 1 bis 7 kultiviert, in dem das Gen, das für die Expression eines Proteins von industriellem Interesse codiert, das Gen ist, das für ein Hirudin oder eine seiner natürlichen oder künstlichen Varianten codiert.

11. Verfahren zur Herstellung von Gamma-Interferon, dadurch gekennzeichnet, daß man auf einem vollständigen Medium einen Stamm nach einem der Ansprüche 1 bis 7 kultiviert, in dem das Gen, das für die Expression eines Proteins von industriellem Interesse codiert, das Gen ist, das für Gamma-Interferon codiert.

**Patentansprüche für folgende Vertragsstaaten : ES, GR**

1. Bakterienstamm, der eine Mutation des Gens dapD enthält und durch einen Plasmid-Vektor transformiert worden ist, der ein intaktes Gen dapD sowie ein Gen, das für ein interessierendes Protein codiert, und die Elemente, die seine Expression in dem Wirtsbakterium gewährleisten, trägt.

2. Bakterienstamm nach Anspruch 1, dadurch gekennzeichnet, daß die Chromosomen-Mutation dapD eine Deletion mindestens eines Teils des Gens dapD ist und daß der Plasmid-Vektor ein intaktes Gen dapD trägt.

3. Bakterienstamm nach Anspruch 2, dadurch gekennzeichnet, daß die Deletion eine Gesamtdeletion des Gens dapD ist.

**4.** Bakterienstamm nach einem der Ansprüche 1 bis 3, dadurch gekennzeichnet, daß der Plasmid-Vektor eine Sequenz trägt, die ihn im monomeren Zustand hält.

**5.** Bakterienstamm nach Anspruch 4, dadurch gekennzeichnet, daß die genannte Sequenz eine Sequenz "cer" ist.

**6.** Bakterienstamm nach einem der Ansprüche 1 bis 5, dadurch gekennzeichnet, daß das Wirts-Bakterium ein Stamm von E. coli ist.

**7.** Bakterienstamm nach einem der Ansprüche 5 oder 6, dadurch gekennzeichnet, daß das Protein von industriellem Interesse ausgewählt wird aus Hirudin, Gamma-Interferon, Brenzkatechin-2,3-Oxygenase und $\alpha$-Antitrypsin.

**8.** Verfahren zur Herstellung eines Stammes nach einem der Ansprüche 1 bis 7, bei dem man einen Stamm, der eine Mutation des Gens dapD enthält, mit einem Plasmid-Vektor transformiert, der ein intaktes Gen dapD sowie ein Gen, das für ein interessierendes Protein codiert, und die Elemente, die seine Expression in dem Wirtsbakterium gewährleisten, trägt.

**9.** Verfahren zur Herstellung eines Proteins von industriellem Interesse, dadurch gekennzeichnet, daß man einen Stamm nach einem der Ansprüche 1 bis 7 kultiviert und das genannte interessierende Protein abtrennt (gewinnt).

**10.** Verfahren zur Herstellung von $C_{2,3}O$, dadurch gekennzeichnet, daß man auf einem vollständigen Medium einen Stamm nach einem der Ansprüche 1 bis 7 kultiviert, in dem das Gen, das für die Expression eines Proteins von industriellem Interesse codiert, das Gen ist, das für $C_{2,3}O$ codiert.

**11.** Verfahren zur Herstellung von Hirudin, dadurch gekennzeichnet, daß man auf einem vollständigen Medium einen Stamm nach einem der Ansprüche 1 bis 7 kultiviert, in dem das Gen, das für die Expression eines Proteins von industriellem Interesse codiert, das Gen ist, das für ein Hirudin oder eine seiner natürlichen oder künstlichen Varianten codiert.

**12.** Verfahren zur Herstellung von Gamma-Interferon, dadurch gekennzeichnet, daß man auf einem vollständigen Medium einen Stamm nach einem der Ansprüche 1 bis 7 kultiviert, in dem das Gen, das für die Expression eines Proteins von industriellem Interesse codiert, das Gen ist, das für Gamma-Interferon codiert.

FIG_1

EcoRI

Poly

pTG 192
2,1 kb

Ori

AmpR

AluI    Dap D gene    AluI

P →    PstI

pTG 192
coupé EcoRI
traité à la klenow
traité à la phosphatase

pDB6 coupé par AluI
isoler la bande de 1,3kb

T₄ DNA ligase

EcoRI°

Dap D gene

Poly

Ori

Pst I

pTG 764
3,4kb

EcoRI

Amp R

# FIG_2

FIG_3

# FIG_4

AluI · Dap D gene · AluI

P → · PstI

pDB6 coupé par
AluI , la bande
a 1,3kb isolé

SmaI · BglII
Poly
Amp R · pTG 192 · Ori
2,1 kb
AhaIII

pTG 192
coupé par SmaI AhaIII,
traitement à la phosphatase

T4 DNA
ligase

PstI
AluI° SmaI° BglII
Poly
pTG 766 · Ori
2,24 kb
Dap D
gene
PstI
AhaIII°
AluI°

PstI
AluI° SmaI° BglII
Poly
PstI · pTG 767 · Ori
2,24 kb
DapD gene
AhaIII°
AluI°

# FIG. 5

pDB6 :

le fragment Hind III – BamH I

FIG-6

FIG-7

pTG47 :

le fragment KpnI – BglII

2.4 kb     1.3 kb     2.8 kb

région          can R          région
5' flanquante                  3' flanquante

## FIG-8

FIG-10

FIG-9A

EP 0 258 118 B1

FIG_9B

FIG-9C

FIG_9D

FIG-11

FIG-12

FIG-13

FIG-14

FIG-15

FIG-16

FIG-17

42